(19) Europäisches Patentamt / European Patent Office / Office européen des brevets

(11) EP 2 445 871 B1

(12) **EUROPÄISCHE PATENTSCHRIFT**

(45) Veröffentlichungstag und Bekanntmachung des Hinweises auf die Patenterteilung:
**05.10.2016 Patentblatt 2016/40**

(21) Anmeldenummer: **10725981.4**

(22) Anmeldetag: **01.06.2010**

(51) Int Cl.:
***C07C 303/06*** *(2006.01)* ***C07C 309/17*** *(2006.01)*
***C07C 69/65*** *(2006.01)* ***C07C 67/08*** *(2006.01)*
***B01F 17/00*** *(2006.01)* ***C11D 1/00*** *(2006.01)*

(86) Internationale Anmeldenummer:
**PCT/EP2010/003328**

(87) Internationale Veröffentlichungsnummer:
**WO 2010/149262 (29.12.2010 Gazette 2010/52)**

(54) **FLUORTENSIDE**

FLUOROSURFACTANTS

TENSIOACTIFS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **26.06.2009 DE 102009030846**

(43) Veröffentlichungstag der Anmeldung:
**02.05.2012 Patentblatt 2012/18**

(73) Patentinhaber: **Merck Patent GmbH**
**64293 Darmstadt (DE)**

(72) Erfinder:
- **HIERSE, Wolfgang**
  **64846 Gross-Zimmern (DE)**
- **CLAUS, Eckhard**
  **60388 Frankfurt am Main (DE)**
- **KRAUSE, Jan**
  **63667 Nidda-Eichelsdorf (DE)**
- **KLEINEIDAM, Melanie**
  **64293 Darmstadt (DE)**
- **SCHELLENBERGER, Steffen**
  **64293 Darmstadt (DE)**
- **BATHE, Andreas**
  **64289 Darmstadt (DE)**

(56) Entgegenhaltungen:
**DE-A1-102006 031 143 US-A- 5 300 394**

- **D. Peter Carlson; Walter Schmiegel: "Fluoropolymer, Organics" Ullmann's Encyclopedia of Industrial Chemistry 2005, XP002601557 DOI: 10.1002/14356007.a11_393 Gefunden im Internet: URL:http://onlinelibrary.wiley.com/doi/10.1002/14356007.a11_393/pdf [gefunden am 2010-09-21]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 445 871 B1

## Beschreibung

Gegenstand der vorliegenden Erfindung sind neue Verbindungen mit fluorierten Endgruppen, deren Verwendung als oberflächenaktive Substanzen und Herstellverfahren für diese Verbindungen.

Fluortenside besitzen eine überragende Fähigkeit zur Senkung der Oberflächenspannung, die beispielsweise bei der Hydrophobisierung von Oberflächen z.B. von Textilien, Papier, Glas, Baustoffe oder Adsorbentien genutzt wird. Außerdem besteht die Möglichkeit, sie als Grenzflächenvermittler bzw. Emulgator oder Viskositätsminderer in Farben, Lacken oder Klebstoffen zu verwenden.

In der Regel enthalten Fluortenside Perfluoralkylsubstituenten, die in der Umwelt durch biologische und/oder andere Oxidationsprozesse zu Perfluoralkylcarbonsäuren (PFCA's) und -sulfonsäuren (PFAS's) abgebaut werden. In den letzten Jahren gab die Anreicherung von Perfluoralkylcarbonsäuren (PFCA's) und Perfluoralkylsulfonsäuren (PFAS's) in der Natur Anlass zur Besorgnis. PFCA's und PFAS's sind hoch-persistente Verbindungen deren langkettige Varianten (mit Perfluoralkylketten von 8 oder mehr Kohlenstoffatomen) ein bioaccumulatives Potential haben. Sie stehen zum Teil in Verdacht gesundheitliche Schäden zu verursachen (G. L. Kennedy, Jr., J. L. Butenhoff, G. W. Olsen, J. C. O'Connor, A. M. Seacat, R. G. Biegel, S. R. Murphy, D. G. Farrar, Critical Review in Toxicology, 2004, 34, 351-384).

Spezielle Anwendungen von Sulfotricarballylate mit ggf. fluorierten Alkylgruppen oder Arylgruppen sind in US 4,988,610 sowie US 6,890,608 beschrieben und in A.R. Pitt et al., Colloids and Surfaces A: Physicochemical and Engineering Aspects, 1996, 114, 321-335; A.R. Pitt, Progr. Colloid Polym. Sci, 1997, 103, 307-317 und Z.-T. Liu et al., Ind. Eng. Chem. Res. 2007, 46, 22-28. US 5,300,394 beschreibt ein Verfahren zur Herstellung einer wässrigen Dispersion unter Verwendung fluorierter Tenside

Von der Firma Omnova werden Polymere vertrieben, deren Seitenketten terminale $CF_3$- oder $C_2F_5$-Gruppen aufweisen. In der Internationalen Patentanmeldung WO 03/010128 werden Perfluoralkyl-substituierte Amine, Säuren, Aminosäuren und Thioethersäuren beschrieben, die eine C3-20-Perfluoralkyl-Gruppe aufweisen.

Aus JP-A-2001/133984 sind oberflächenaktive Verbindungen mit Perfluoralkoxy-Ketten bekannt, die sich zum Einsatz in Antireflex-Beschichtungen eignen. Aus JP-A-09/111286 ist die Verwendung von Perfluorpolyethertensiden in Emulsionen bekannt. In der DE 102005000858 A1 werden Verbindungen, die mindestens eine endständige Pentafluorsulfuranyl-Gruppe oder mindestens eine endständige Trifluormethoxy-Gruppe tragen und über eine polare Endgruppe verfügen, oberflächenaktiv sind und sich als Tenside eignen beschrieben.

Aus US5300394 sind Fluortenside bekannt.

Weiterhin besteht Bedarf nach alternativen oberflächenaktiven Substanzen vorzugsweise mit einem, den klassischen Fluortensiden, vergleichbaren Eigenschaftsprofil, sowie einer ebenso großen chemischen Vielseitigkeit, die vorzugsweise beim oxidativen oder reduktiven Abbau nicht zu langkettigen persistenten Fluorcarbon- bzw. Fluorsulfonsäuren abbauen oder vorzugsweise in geringerer Dosierung effektiv sind als herkömmliche Fluortenside.

Es wurden nun neue Verbindungen gefunden, die als oberflächenaktive Substanzen geeignet sind und bevorzugt einen oder mehrere der o. g. Nachteile nicht aufweisen.

Ein erster Gegenstand der vorliegenden Erfindung sind Verbindungen der Formel (I)

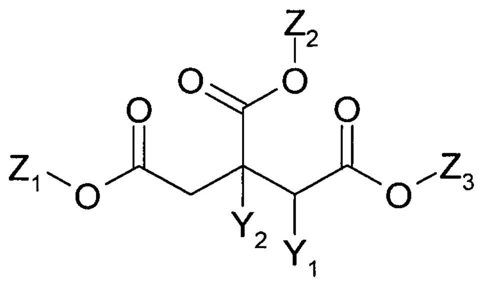

X

(I)

wobei

die Gruppen $Z_i$ ($Z_1$, $Z_2$ und $Z_3$) unabhängig voneinander verzweigte oder unverzweigte Alkylgruppen oder Gruppen der Struktur $R_i(A(CR^1R^2)_{ci}(CR^3R^4)_{c'i})_{di}$- mit den jeweiligen Indizes ci und c'i unabhängig voneinander = 0-10 und di = 1-5 sind, wobei $R_i$ ein verzweigter oder unverzweigter, fluorhaltiger Alkylrest ist, $R^1$ bis $R^4$ unabhängig voneinander Wasserstoff oder eine verzweigte oder unverzweigte Alkylgruppe sind, ci und c'i nicht gleichzeitig = 0 sind und A = O, S und /oder N ist,

$Y_1$ eine anionische polare Gruppe und $Y_2$ ein Wasserstoffatom ist oder umgekehrt,

X ein Kation ist

und mindestens eine der Gruppen $Z_i$ eine Gruppe der Struktur $R_i(A(CR^1R^2)_{ci}(CR^3R^4)_{c'i})_{di}$- ist.

Für Formel (I) gilt bevorzugt, dass a) di > 0 ist wenn $Z_1$, $Z_2$ und $Z_3$ alle eine Gruppe der Struktur $R_i(O(CH_2)_{ci})_{di}$ sind und alle $R_i$ ausgewählt sind aus $CF_3CF_2CH_2$-, $CF_3CF_2CH_2CH_2$-, $CF_3CF_2CF_2CH_2$- oder $H(CF_2)_4CH_2$-und/oder dass b) $Y_2$ nicht gleich $OSO_3^-$ ist und X nicht gleich $Na^+$ oder $K^+$ ist, wenn $Z_1 = Z_2 = Z_3$ und alle $Z_i$ ausgewählt sind aus $C_4F_9CH_2CH_2$-, $C_6F_{13}CH_2CH_2$- oder $C_8F_{17}CH_2CH_2$-.

Die Reste $R_i$ sind verzweigte oder unverzweigte, fluorhaltige Alkylgruppen. Die Reste $R_i$ können teil- oder perfluoriert sein und weisen bevorzugt terminale perfluorierte Gruppen auf. Bevorzugt sind verzweigte oder unverzweigte, fluorhaltige Alkylgruppen mit 1 bis 10 C-Atomen.

Unverzweigte fluorhaltige Alkylgruppen enthalten bevorzugt 1 bis 6 C-Atomen, insbesondere 1 - 4 C-Atome. Verzweigte fluorhaltige Alkylgruppen enthalten bevorzugt 3 bis 6 C-Atome, insbesondere 3 - 4 C-Atome. Als verzweigte fluorhaltige Alkylgruppen werden bevorzugt $(CF_3)_2$-CH- oder $(CF_3)_3$-C- Gruppen verwendet.

$R^1$ bis $R^4$ sind bevorzugt unabhängig voneinander Wasserstoff oder eine $C_{1-4}$-Alkylgruppe, insbesondere H oder $CH_3$. ci und c'i sind bevorzugt unabhängig voneinander eine ganze Zahl aus dem Bereich von 0 bis 6, insbesondere von 0 - 3, besonders bevorzugt gleich 0 - 2, wobei ci und c'i nicht gleichzeitig = 0 sind. di ist bevorzugt eine ganze Zahl aus dem Bereich von 1 - 3, insbesondere 1.

A ist bevorzugt gleich O oder S, insbesondere gleich O. Insbesondere sind die Gruppen $Z_i$ gleich $R_i(O(CR^1R^2)_{ci}(CR^3R^4)_{c'i})_{di}$-.

Insbesondere bevorzugt sind Verbindungen mit $R^1$ bis $R^3$ gleich Wasserstoff, $R^4$ gleich Wasserstoff oder Methyl, ci und c'i unabhängig voneinander gleich 0-2, insbesondere 1, di gleich 1-3, insbesondere 1-3, und A gleich O, wobei ci und c'i nicht gleichzeitig = 0 sind. In einer bevorzugten Variante der Erfindung ist $R^4$ dabei gleich Methyl und die Variablen haben diese bevorzugten Bedeutungen. Geeignet sind auch Verbindungen in denen $R^1$ gleich Methyl und $R^2$ bis $R^4$ gleich Wasserstoff sind, wobei die Variablen die bevorzugten Bedeutungen haben.

Besonders bevorzugt sind Verbindungen der Formel (I), in denen die fluorhaltigen Gruppen $Z_i$ gleich $R_i(O(CH_2)_{ci})_{di}$- mit den jeweiligen Indizes ci=2-10, bevorzugt 2-4, insbesondere gleich 2, und di=1-5, bevorzugt 0-3, besonders bevorzugt 1-3, insbesondere gleich 1, $Y_1$ eine anionische polare Gruppe und $Y_2$ ein Wasserstoffatom ist oder umgekehrt, X ein Kation ist und mindestens eine der Gruppen $Z_i$ eine Gruppe der Struktur $R_i(O(CH_2)_{ci})_{di}$- ist, wobei bevorzugt a) d > 0 ist, wenn $Z_1$, $Z_2$ und $Z_3$ alle eine Gruppe der Struktur $R_i(O(CH_2)_{ci})_{di}$- sind und alle $R_i$ ausgewählt sind aus $CF_3CF_2CH_2$-, $CF_3CF_2CH_2CH_2$-, $CF_3CF_2CF_2CH_2$- oder $H(CF_2)_4CH_2$- und b) $Y_2$ nicht gleich $OSO_3^-$ und X nicht gleich $Na^+$ oder $K^+$ ist, wenn $Z_1 = Z_2 = Z_3$ und alle $Z_i$ ausgewählt sind aus $C_4F_9CH_2CH_2$-, $C_6F_{13}CH_2CH_2$- oder $C_8F_{17}CH_2CH_2$-.

Weiter bevorzugt sind Verbindungen der Formel (I), in denen $Z_1$, $Z_2$ und $Z_3$ unabhängig voneinander $F_3C(CF_2)_{ai}(CH_2)_{bi}(O(CH_2)_{ci})_{di}$-Gruppen mit ai=0-6, bevorzugt gleich 1-5, insbesondere gleich 1-2, bi=1-6, bevorzugt gleich 1-3, insbesondere gleich 1-2, ci=2-10, bevorzugt gleich 2-4, insbesondere gleich 2, und di=1-5, bevorzugt gleich 1-3, insbesondere gleich 1, sind, wobei insbesondere $Z_1$=$Z_2$=$Z_3$ sind. Bevorzugt sind auch Verbindungen, in denen ai = 0, 1 oder 2, bevorzugt gleich 1 bis 2, insbesondere gleich 1, $b_i$ = 1, $_{Ci}$ = 2 und $d_i$ = 1 oder 2, insbesondere gleich 1, ist und wobei $a_1 + a_2 + a_3 > 1$ ist.

Wenn alle $Z_1$, $Z_2$ und $Z_3$ ausgewählt sind aus $CF_3CF_2CH_2$-, $CF_3CF_2CH_2CH_2$-, $CF_3CF_2CF_2CH_2$- oder $H(CF_2)_4CH_2$-, ist di in Formel (I) bevorzugt größer 0. Wenn $Z_1 = Z_2 = Z_3$ und alle $Z_i$ ausgewählt sind aus $C_4F_9CH_2CH_2$-, $C_6F_{13}CH_2CH_2$- oder $C_8F_{17}CH_2CH_2$-, ist $Y_2$ nicht gleich $OSO_3^-$und X nicht gleich $Na^+$ oder $K^+$.

In einer Ausgestaltung der Erfindung können die erfindungsgemäßen Verbindungen als Gemische vorliegen, in denen die einzelnen Verbindungen unterschiedliche Werte für ci, c'i und/oder di besitzen.

Die erfindungsgemäßen Verbindungen der Formel (I) können eine oder mehrere erfindungsgemäße fluorierte Gruppen $Z_i$ enthalten. Bevorzugt enthalten die Verbindungen zwei oder drei fluorierte Gruppen $Z_i$, insbesondere solche mit drei fluorierten Gruppen $Z_i$ sind bevorzugt.

Die drei Substituenten $Z_1$, $Z_2$ und $Z_3$ können alle gleich, alle verschieden oder zwei von ihnen gleich und einer davon verschieden sein, unter der Voraussetzung, dass $Z_1$, $Z_2$ und $Z_3$ nicht alle gleichzeitig nichtfluorierte, verzweigte oder unverzweigte Alkylreste sind. Bevorzugte Verbindungen sind solche, in denen zwei von $Z_1$, $Z_2$ und $Z_3$ oder alle $Z_1$, $Z_2$ und $Z_3$ gleich sind, insbesondere gleich den beschriebenen bevorzugten $R_i(O(CR^1R^2)_{ci}(CR^3R^4)_{c'i})_{di}$-Gruppen. Ganz besonders bevorzugt sind hierbei folgende fluorhaltige Alkylreste: $CF_3CH_2$-, $CF_3CF_2CH_2$-, $CF_3CF_2CF_2CH_2$-, $CF_3CF_2CH_2CH_2$-, $CF_3CF_2CH_2CH_2CH_2$-, $CF_3CFHCF_2CH_2$-, $(CF_3)_2CH$-, $(CF_3)_3C$-, $CF_3(CF_2)_3CH_2CH_2$-; $CF_3(CF_2)_5CH_2CH_2$-; $CF_3(CF_2)_4CH_2$-. Bevorzugt umfassen die Verbindungen fluorhaltige Alkylreste mit höchstens 3 fluortragenden Kohlenstoffatomen. Besonders bevorzugte Verbindungen der Formel (I) mit $Z_1 = Z_2 = Z_3$ sind solche, in denen ci bzw. c'i gleich 0 bis 3, insbesondere 0-2, und di > 0 ist, wobei ci und c'i nicht gleichzeitig = 0 sind. Vor allem erfindungsgemäße Verbindungen mit di gleich 1 bis 3, insbesondere gleich 1, sind bevorzugt.

Enthalten die Verbindungen nichtfluorierte Endgruppen $Z_1$, $Z_2$ und $Z_3$, so sind diese bevorzugt unabhängig voneinander lineares oder verzweigtes Alkyl mit 1 bis 20 C-Atomen, bevorzugt 1 bis 10 C-Atomen. Insbesondere sind $Z_1$, $Z_2$ und $Z_3$ unabhängig voneinander lineares Alkyl mit 3 bis 10 C-Atomen, besonders bevorzugt mit 3 bis 8 C-Atomen. Sind zwei der Gruppen $Z_1$, $Z_2$ und $Z_3$ nichtfluoriert, so sind bevorzugt diese beiden Gruppen gleich.

In einer bevorzugten Gruppe von erfindungsgemäß einzusetzenden Verbindungen bzw. erfindungsgemäßen Verbindungen stehen $Y_1$ oder $Y_2$, wobei eines von beiden gleich Wasserstoffatom ist, für eine anionische polare Gruppe ausgewählt aus $-COO^-$, $-SO_3^-$, $-OSO_3^-$, $-PO_3^{2-}$, $-OPO_3^{2-}$,$-(OCH_2CH_2)_s-O-(CH_2)_t-COO^-$, $-(OCH_2CH_2)_s-O-(CH_2)_t$ $-SO_3^-$ ,$-(OCH_2CH_2)_s-O-(CH_2)_t-OSO_3^-$, $-(OCH_2CH_2)_s-O-(CH_2)_t-PO_3^{2-}$, $-(OCH_2CH_2)_s-O-(CH_2)_t-OPO_3^{2-}$ oder für die Formeln A bis C,

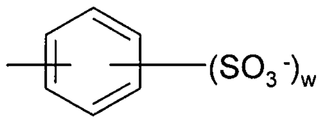

A

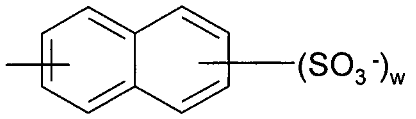

B

oder

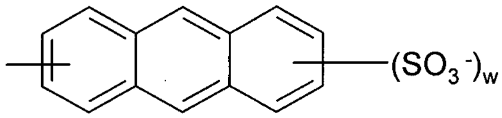

C

wobei s steht für eine ganze Zahl aus dem Bereich von 1 bis 1000, t steht für eine ganze Zahl ausgewählt aus 1, 2, 3 oder 4 und w steht für eine ganze Zahl ausgewählt aus 1, 2 oder 3.

Zu den bevorzugten anionischen Gruppen gehören dabei insbesondere $-COO^-$, $-SO_3^-$, $-OSO_3^-$, $-PO_3^{2-}$, $-OPO_3^{2-}$ , die Teilformel A, sowie $-(OCH_2CH_2)_s-O-(CH_2)_t-COO^-$, $-(OCH_2CH_2)_s-O-(CH_2)_t-SO_3^-$ und$-(OCH_2CH_2)_s-O-(CH_2)_t-OSO_3^-$ , wobei jede einzelne dieser Gruppen für sich genommen bevorzugt sein kann.

Zu den ganz besonders bevorzugten anionischen Gruppen gehören dabei $-SO_3^-$, $-OSO_3^-$, $-PO_3^{2-}$, oder $OPO_3^{2-}$ , insbesondere $-SO_3^-$. Bevorzugt sind insbesondere Verbindungen in denen $Y_1$ eine Sulfonatgruppe $-SO_3^-$ und $Y_2$ ein Wasserstoffatom ist.

Bevorzugt ist X ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt ist $X = Na^+$, $K^+$ oder $NH_4^+$, besonders bevorzugt $Na^+$.

Bevorzugte Verbindungen der Formel (I) sind insbesondere solche Verbindungen, in denen alle Variablen die bevorzugten Bedeutungen haben. Explizit solche Verbindungen sind bevorzugt, in denen $Z_1=Z_2=Z_3=$ $F_3C(CF_2)_{ai}(CH_2)_{bi}(O(CH_2)_{ci})_{di}$ mit ai=1-2, bi=1-2, ci=2, di=1-3, $Y_1$ oder $Y_2$ eine Sulfonatgruppe $-SO_3^-$ und $X=Na^+$ ist.

Besonders bevorzugt sind Verbindungen der Formel (Ia) oder (Ib)

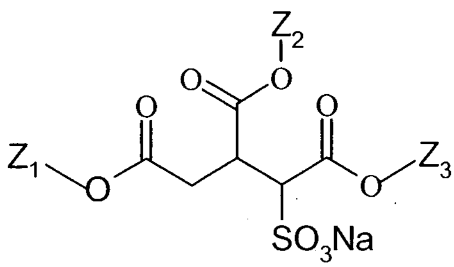

(Ia)

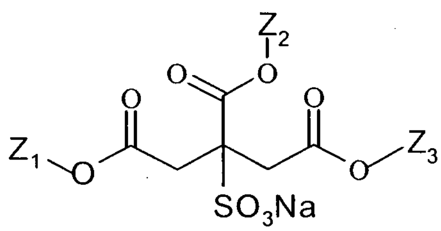

(Ib)

wobei $Z_1=Z_2=Z_3$ und alle $Z_i$ ausgewählt sind aus $R_j(O(CH_2)_{ci})_{di}$ mit ci=2, di=1-3 und $R_j$ = $CF_3CF_2CH_2$- oder $CF_3CF_2CF_2CH_2$-.

Die erfindungsgemäßen Verbindungen der Formel (I) werden in der vorliegenden Erfindung als Sulfotricarballylate bezeichnet und basieren auf Estern der Aconitsäure, an deren Doppelbindung eine Sulfonatgruppe addiert wurde. Insbesondere Sulfotricarballylate mit drei erfindungsgemäßen fluorierten Endgruppen sind bevorzugt. Die erfindungsgemäßen Verbindungen gemäß Formel (I) können auch als Gemische vorliegen, besonders auch als Isomerengemische (Konstitutions- und/oder Konfigurationsisomerengemische). Insbesondere Diastereomeren- und/oder Enantiomerengemische sind möglich.

Beispiele bevorzugter erfindungsgemäßer Verbindungen sind:

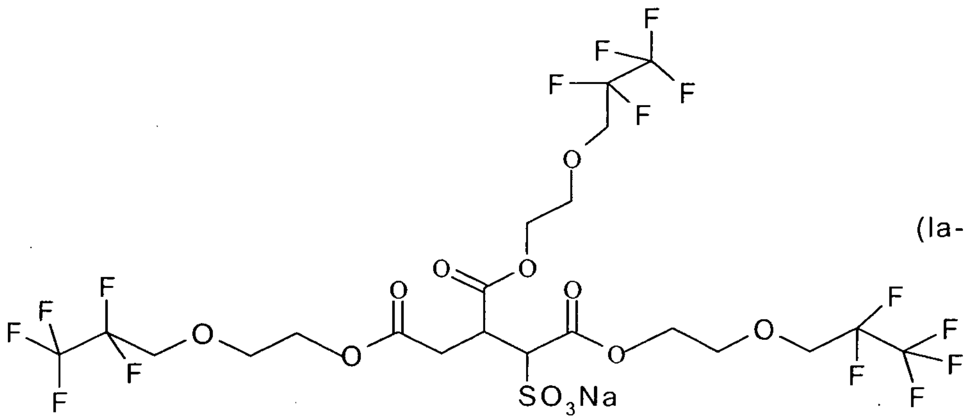

(Ia-1)

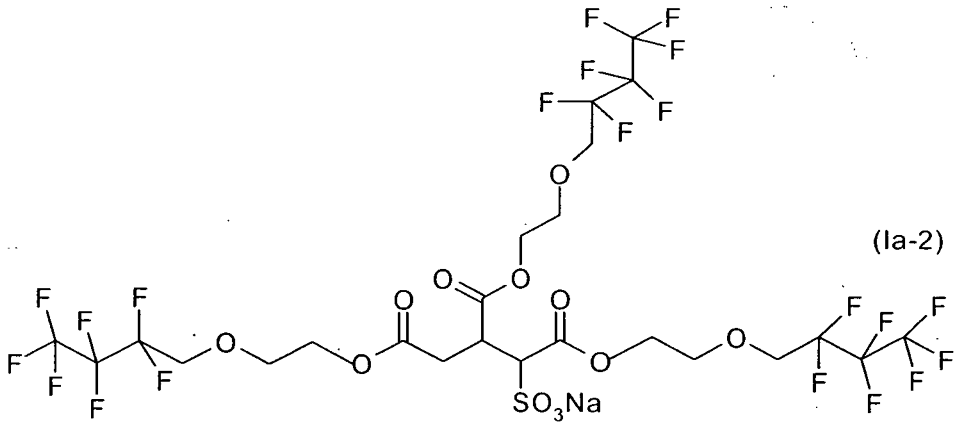

(Ia-2)

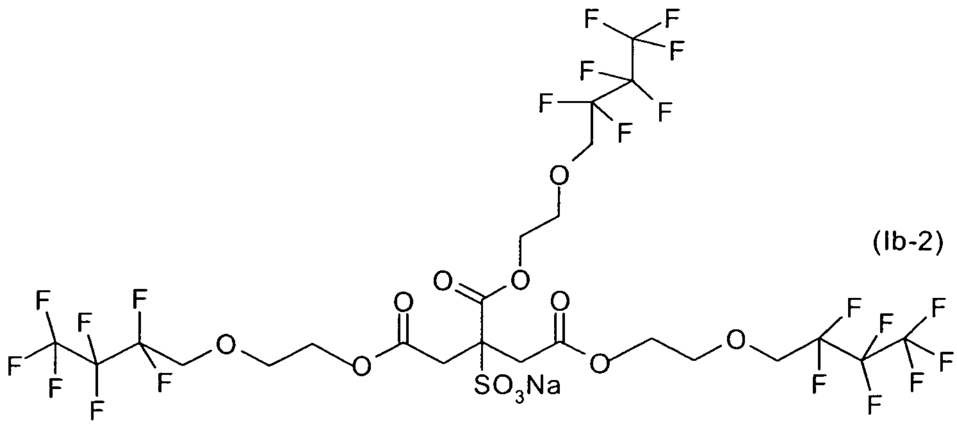

(Ib-2)

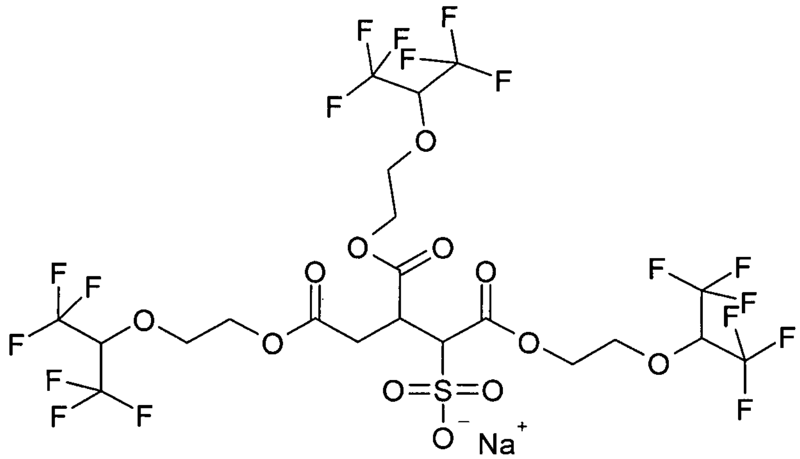

(Ia-3)

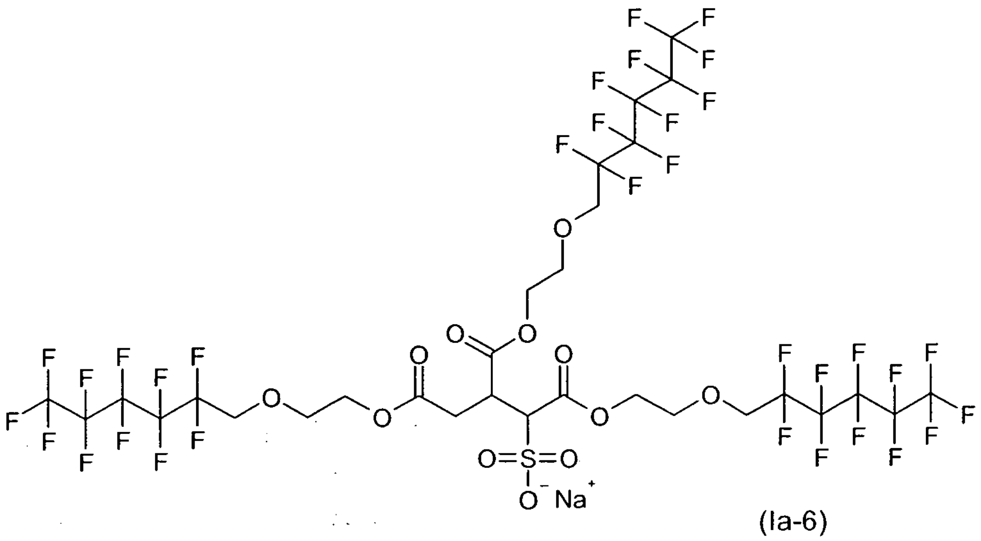

(Ia-6)

Wobei Verbindungen der Formeln Ia-1 und Ia-2 besonders bevorzugt sind.

Vorteile der erfindungsgemäßen Verbindungen können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),
- schwache Schaumwirkung und/oder geringe Schaumstabilisierung,
- gute Verarbeitbarkeit in Formulierungen und/oder
- Lagerstabilität.

Bevorzugt weisen die erfindungsgemäßen Verbindungen eine besondere Oberflächenaktivität auf. Die erfindungsgemäßen Verbindungen der Formel (I) können gegenüber den Fluortensiden des Standes der Technik deutlich verbesserte Umwelteigenschaften aufweisen, da sie weder chemisch noch biologisch zu langkettigen PFCAs oder PFASs abbauen.

Ein zweiter Gegenstand der vorliegenden Erfindung ist die Verwendung der erfindungsgemäßen Verbindungen, insbesondere der bevorzugten Verbindungen der Formel (I), als oberflächenaktive Mittel, beispielsweise zur Verbesserung des Verlaufsverhaltens und des Benetzungsvermögens von Coatingformulierungen.

Dabei können die im Vorangegangenen beschriebenen bevorzugten Ausgestaltungen der erfindungsgemäßen Verbindungen besonders vorteilhaft verwendet werden. Sulfotricarballylate, die zwei oder drei, insbesondere drei, erfindungsgemäße fluorierte Gruppen enthalten, werden bevorzugt verwendet. Bevorzugt werden Verbindungen der Formeln (Ia) und/oder (Ib) verwendet. Die erfindungsgemäßen Verbindungen können auch als Gemische, besonders auch

als Isomerengemische (Konstitutions- und/oder Konfigurationsisomerengemische) verwendet werden. Insbesondere Diastereomeren- und/oder Enantiomerengemische sind möglich.

Einsatzgebiete sind beispielsweise die Verwendung der erfindungsgemäßen Verbindungen als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Poliermitteln und Wachsen (insbesondere für Mobiliar, Fußböden und Automobile), oder in Additivzubereitungen zur Additivierung entsprechender Zubereitungen.

Dabei werden die erfindungsgemäßen Verbindungen für die Anwendung üblicherweise in entsprechend ausgelegten Zubereitungen eingebracht. Entsprechende Mittel, enthaltend mindestens eine erfindungsgemäße Verbindung, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bei bevorzugten Mitteln handelt es sich dabei um Farb- und Lackzubereitungen sowie Druckfarben.

Insbesondere sind auch wasserbasierte Lackformulierungen, die mindestens eine der erfindungsgemäßen Verbindungen allein oder im Gemische mit anderen Tenside enthalten, Gegenstand der vorliegenden Erfindung. Bevorzugt werden Lackformulierungen auf Basis der folgenden synthetischen Filmbildner verwendet:

- Polykondensationsharze wie Alkydharze, gesättigt/ungesättigte Polyester,
- Polyamide/imide, Silikonharze; Phenolharze; Harnstoffharze und Melaminharze,
- Polyadditionsharze wie Polyurethane und Epoxidharze,
- Polymerisationsharze wie Polyolefine, Polyvinylverbindungen und Polyacrylate.

Außerdem sind die erfindungsgemäßen Verbindungen auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack. Die erfindungsgemäßen Verbindungen können sowohl in physikalisch härtenden (Thermoplaste) als auch in vernetzenden (Elastomere und Duromere) wässrigen Lacksystemen verwendet werden. Bevorzugt verbessern die erfindungsgemäßen Verbindungen die Verlaufs- und Benetzungseigenschaften der Lacksysteme.

Ein weiterer Einsatzbereich der erfindungsgemäßen Verbindungen liegt in Herstellprozessen von Polymeren, insbesondere von Fluorpolymeren. Wichtige technische Methoden zur Herstellung von Fluorpolymeren wie z. B. Polytetrafluorethylen (PTFE) sind z. B. Emulsions- und Suspensionspolymerisation. Suspension- und Emulsionspolymerisationsverfahren sind gängige, dem Fachmann wohl bekannte Polymerisationsverfahren. Bei Suspension- und Emulsionspolymerisationsverfahren enthält das System immer mindestens vier Bestandteile: (überwiegend) wasserunlösliches Monomer, Wasser, Dispergiermittel bzw. Emulgiermittel und Initiator. Die Durchführung der genannten Polymerisationsverfahren ist dem Fachmann geläufig. Bei diesem Prozess wird das Polymer in einem Autoklaven, der Wasser, das oder die entsprechenden, meist gasförmigen, Monomere, Initiatoren, Tenside und andere Hilfsmittel enthält, unter Rühren und ständiger Temperatur- und Druckkontrolle hergestellt. Die erfindungsgemäßen Verbindungen sind geeignet, als Tenside die sehr hydrophoben Fluorpolymertropfen bzw. -teilchen in der wässrigen Lösung dispergiert zu halten.

Außerdem können die erfindungsgemäßen Verbindungen in Hydrophobiermitteln, Oleophobiermitteln, Netz-/Verlaufsmitteln, Schutz-/Reinigungsmitteln gegen Flecken und Verschmutzungen, Stain Releases, Beschlagschutzmitteln, Gleitmitteln, Entschäumern, Entlüftern, Trocknungsbeschleunigern, Verbesserern der Abriebfestigkeit und mechanischen Strapazierfähigkeit, und Antistatika, insbesondere in der Behandlung von Textilien (insbesondere Bekleidung, Teppiche und Teppichböden, Polsterbezüge in Mobiliar und Automobilen) und harte Oberflächen (insbesondere Küchenoberflächen, Sanitäranlagen, Kacheln, Glas), nicht-gewobenen textilen Werkstoffen, Lederwaren, Papieren und Kartonnagen, Holz und holzbasierten Werkstoffen, mineralischen Substraten wie Stein, Zement, Beton, Gips, Keramiken (glasierte und unglasierte Ziegel, Steingut, Porzellan) und Gläsern, sowie für Kunststoffe und metallische Substrate eingesetzt werden. Für metallische Substrate ist zusätzlich auch die Verwendung in Korrosionsschutzmitteln Gegenstand der Ansprüche. Für Kunststoffe und Formen für die Kunststoffverarbeitung ist zusätzlich auch die Verwendung in Formtrennmitteln Gegenstand der Ansprüche. Im Falle der Reinigungsmittel und Fleckentferner ist zusätzlich auch die Verwendung als Detergenz bzw. Schmutzemulgier- und - dispergiermittel Gegenstand der Ansprüche.

Weiterhin können die erfindungsgemäßen Verbindungen als antimikrobieller Wirkstoff, insbesondere als Reagenz für die antimikrobielle Oberflächenmodifikation, verwendet werden.

Ein weiterer Einsatzbereich der erfindungsgemäßen Verbindungen liegt in Additiven oder in Additivzubereitungen in Druckfarben, mit einer oder mehreren der folgenden Funktionen: Entschäumer, Entlüfter, Mittel zur Reibungskontrolle, Netzmittel, Verlaufsmittel, Verbesserer der Pigmentkompatibilität, Verbesserer der Druckauflösung, Trocknungsbeschleuniger.

Die erfindungsgemäßen Verbindungen können auch als Schaumstabilisatoren und/oder zur Unterstützung der Filmbildung, insbesondere in wässrigen filmbildenden Feuerlöschschäumen, sowohl synthetisch als auch proteinbasiert,

auch für alkoholresistente Formulierungen (AFFF und AFFF-AR, FP, FFFP und FFFP-AR) verwendet werden.

Weiterhin können die erfindungsgemäßen Verbindungen als Additive in polymeren Werkstoffen (Kunststoffen), mit einer oder mehreren der folgenden Funktionen eingesetzt werden: Gleitmittel, Verminderer der inneren Reibung, UV-Stabilisator, Hydrophobiermittel, Oleophobiermittel, Schutzmittel gegen Flecken und Verschmutzungen, Kupplungsmittel für Füllstoffe, Flammschutzmittel, Migrationsinhibitor (insbesondere gegen Migration von Weichmachern), Beschlagschutzmittel.

Außerdem sind die erfindungsgemäßen Verbindungen auch zum Einsatz als Additive in flüssigen Medien zur Reinigung, zum Ätzen, zur reaktiven Modifikation und/oder Substanzabscheidung auf Metalloberflächen (insbesondere auch Galvanik und Eloxierung) oder Halbleiteroberflächen (insbesondere für die Halbleiter-Photolithographie: Entwickler, Stripper, Edge Bead Remover, Ätz- und Reinigungsmittel), als Netzmittel und/oder Verbesserer der Qualität von abgeschiedenen Filmen geeignet.

Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Für die erfindungsgemäßen Verbindungen besteht weiterhin die Verwendung als Additive in Herbiziden, Pestiziden und Fungiziden, mit einer oder mehreren der folgenden Funktionen: Substratnetzmittel, Adjuvans, Schauminhibitor, Dispergiermittel, Emulsionsstabilisator. Zusätzlich können die erfindungsgemäßen Verbindungen auch als Additive in Enteisungsmitteln oder Vereisungsverhinderern verwendet werden.

Darüber hinaus eignen sich die erfindungsgemäßen Verbindungen als Additive in Zubereitungen zur Erzaufbereitung, insbesondere Flotations- und Auslaugungslösungen, mit einer oder mehreren der folgenden Funktionen: Netzmittel, Schaummittel, Schauminhibitor, sowie als Additive in Mitteln zur Stimulation von Erdölquellen, mit einer oder mehreren der folgenden Funktionen: Netzmittel, Schaummittel, Emulgator.

Ein weiterer Einsatzbereich der erfindungsgemäßen Verbindungen liegt in der Verwendung als Additive in Klebstoffen, mit einer oder mehreren der folgenden Funktionen: Netzmittel, Penetrationsmittel, Substrat-Haftverbesserer, Entschäumer.

Weiterhin können die erfindungsgemäßen Verbindungen als Additive in Schmierstoffen und Hydraulikflüssigkeiten, mit einer oder mehreren der folgenden Funktionen eingesetzt werden: Netzmittel, Korrosionsinhibitor. Im Falle der Schmierstoffe ist zusätzlich auch die Verwendung als Dispergiermittel (insbesondere für Fluorpolymerpartikel) Gegenstand der Ansprüche.

Auch können die erfindungsgemäßen Verbindungen als Additive in Kitt- und Füllmassen, mit einer oder mehreren der folgenden Funktionen verwendet werden: Hydrophobiermittel, Oleophobiermittel, Schutzmittel gegen Verschmutzungen, Verbesserer der Witterungsbeständigkeit, UV-Stabilisator, Mittel gegen Silikon-Ausblutung.

Alle hier genannten Verwendungen erfindungsgemäß einzusetzender Verbindungen sind Gegenstand der vorliegenden Erfindung. Die jeweilige Anwendung von Tensiden zu den genannten Zwecken ist dem Fachmann bekannt, so dass der Einsatz der erfindungsgemäß einzusetzenden Verbindungen keine Probleme bereitet. Bevorzugt werden die erfindungsgemäßen Verbindungen der Formel (I) in Farb- und Lackzubereitungen, Druckfarben und Wachsen, sowie Poliermitteln verwendet. Insbesondere bevorzugt ist die Verwendung in Farb- und Lackzubereitungen.

Besonders vorteilhaft ist die Anwendung in Farb- und Lackzubereitungen von Verbindungen der Formel (I), insbesondere der im Vorangegangenen als bevorzugt beschriebenen Verbindungen der Formel (I). Verbindungen, in denen die Gruppen $Z_i$ gleich $R_i(O(CH_2)_{ci})_{di}$- mit den jeweiligen Indizes ci=2-10, bevorzugt 2-4, insbesondere gleich 2, und di=1-5, bevorzugt 1-3, insbesondere gleich 1, sind hier bevorzugt. Verbindungen der Formel (I), in denen $Z_1$=$Z_2$=$Z_3$= $F_3C(CF_2)_{ai}(CH_2)_{bi}(O(CH_2)_{ci})_{di}$ ist mit ai=0-6, bevorzugt 1-5, insbesondere 1-2, bi=1-6, bevorzugt 1-3, insbesondere 1-2, ci=2, di=1-3, bevorzugt 1, $Y_1$ oder $Y_2$ gleich eine Sulfonatgruppe $-SO_3^-$ und $X=Na^+$ ist, sind besonders bevorzugt. Insbesondere Verbindungen der Formeln Ia-1 bis Ia-6 eignen sich für die Anwendung in Farb- und Lackzubereitungen, besonders die der Formeln Ia-1 und Ia-2.

Die erfindungsgemäßen Verbindungen der Formel (I) können bevorzugt durch Veresterung von Aconitsäure bzw. dem Anhydrid oder Säurechlorid oder von Zitronensäure mit einem oder mehreren Alkoholen $Z_iOH$ (II) und anschließende Addition, bevorzugt von Natriumhydrogensulfit, hergestellt werden.

$Z_i$ ist hierbei eine nichtfluorierte, verzweigte oder unverzweigte, Alkylgruppe oder eine Gruppe der Struktur $R_i(A(CR^1R^2)_{ci}(CR^3R^4)_{c'i})_{di}$- mit den jeweiligen Indizes ci und c'i unabhängig voneinander = 0-10 und di = 1-5 sind, wobei $R_i$ ein verzweigter oder unverzweigter, fluorhaltiger Alkylrest ist, $R^1$ bis $R^4$ unabhängig voneinander Wasserstoff oder eine verzweigte oder unverzweigte Alkylgruppe sind, ci und c'i nicht gleichzeitig = 0 sind und A = O, S und /oder N ist. Bevorzugt umfasst $Z_i$ die für die bevorzugten Verbindungen der Formel (I) beschriebenen Variablen, insbesondere eine $F_3C(CF_2)_{ai}(CH_2)_{bi}(O(CH_2)_{ci})_{di}$-Gruppe, mit ai=0-6, bi=1-6, ci=2-10 und di=15. ai ist bevorzugt gleich 1 oder 2, bi ist bevorzugt gleich 1 bis 3, insbesondere 1 bis 2. ci ist bevorzugt gleich 2 bis 4, insbesondere 2. di ist bevorzugt gleich 1 bis 3, insbesondere 1.

Bevorzugt werden Alkohole mit $F_3C(CF_2)_{ai}(CH_2)_{bi}(O(CH_2)_{ci})_{di}$-Gruppen verwendet, wobei einer oder auch verschiedene Alkohole im Gemisch oder stufenweise umgesetzt werden können. Bevorzugt wird insbesondere nur ein Alkohol verwendet.

Die Veresterung kann auch im Gemisch mit nichtfluorierten, verzweigten oder unverzweigten Alkoholen erfolgen oder stufenweise mit verschiedenen Alkoholen durchgeführt werden. Die nichtfluorierten, verzweigten oder unverzweigten Alkohole sind bevorzugt solche mit linearem oder verzweigtem Alkyl mit 1 bis 20 C-Atomen, bevorzugt 1 bis 10 C-Atomen. Insbesondere bevorzugt sind Alkohole mit 3 bis 10 C-Atomen, besonders bevorzugt mit 3 bis 8 C-Atomen.

Ein dritter Gegenstand der vorliegenden Erfindung ist somit ein Verfahren zur Herstellung von Verbindungen der Formel (I), insbesondere von Sulfotricarballylaten, die zwei oder drei erfindungsgemäß fluorierte Endgruppen enthalten.

Die verwendeten Alkohole sind kommerziell erhältlich und/oder ihre Herstellung ist dem Fachmann geläufig (Heilmann et al. J. Fluorine Chem. 1992, 59, 387; Janulis et al. US 5,157,159 (1992)).

Die Synthese der Aconitester erfolgt bevorzugt in Gegenwart eines üblichen Katalysators, wie z. B. Toluol-4-sulfonsäure-Monohydrat:

$Z_1OH$

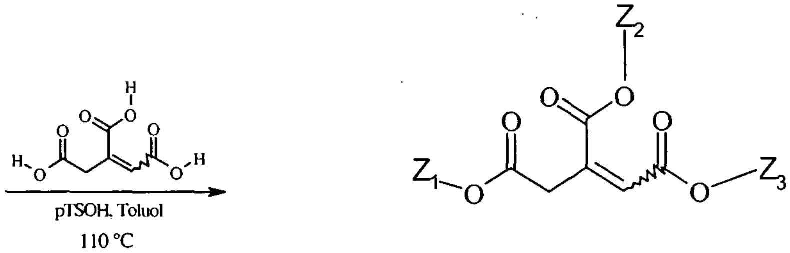


(II) (III)

Weiterhin kann die Synthese der Aconitester bevorzugt ausgehend von Zitronensäure in Gegenwart eines üblichen Katalysators, wie z. B. Schwefelsäure, erfolgen. Auch die Herstellung der entsprechenden Zitronensäureester (IV) ist möglich.

$Z_1OH$

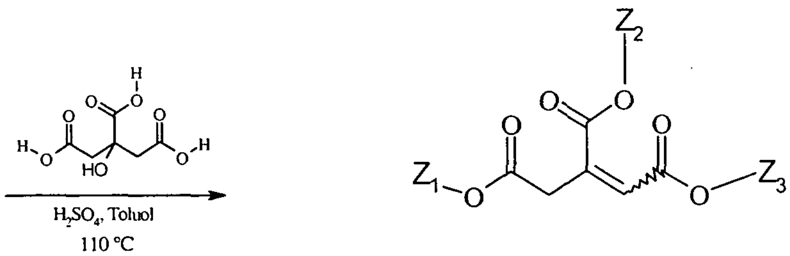


(II) (III)

$Z_1OH$

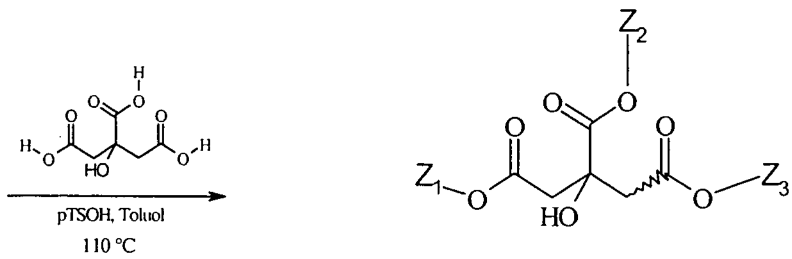


(II) (IV)

In einem zweiten Schritt erfolgt dann die Einführung der Gruppe Y durch Addition an die Doppelbindung der Aconitester bzw. Derivatisierung der OH-Gruppe der Zitronensäureester nach dem Fachmann geläufigen Methoden. Das folgende Schema zeigt beispielhaft die Synthese der Sulfotricarballylate durch die Addition von Natriumhydrogensulfit, die unter dem Fachmann bekannten Bedingungen erfolgen kann:

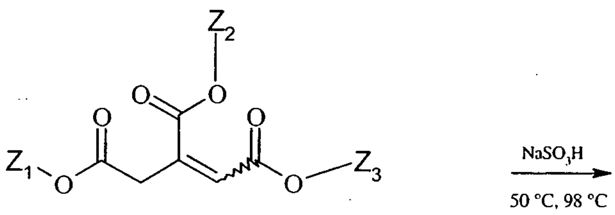


(III)

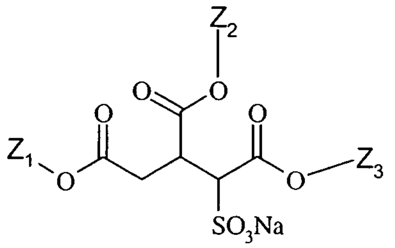

(V)

und/oder

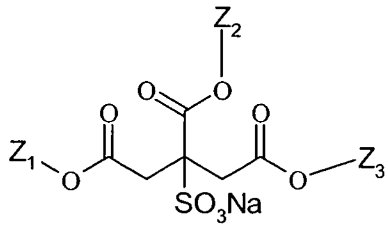

(VI)

Die Formel (III) gibt das Vorliegen von Z/E-Doppelbindungs-Isomeren wieder. Die Herstellung weiterer erfindungsgemäßer Verbindungen kann analog zu den oben gezeigten beispielhaften Reaktionen erfolgen. Die Herstellung weiterer erfindungsgemäßer Verbindungen kann auch nach anderen dem Fachmann an sich aus der Literatur bekannten Methoden erfolgen. Insbesondere andere Veresterungskatalysatoren können verwendet werden.

Ein vierter Gegenstand der vorliegenden Erfindung sind Verbindungen der Formel (III), die als Zwischenstufen in den oben beschriebenen Synthesen der erfindungsgemäßen Verbindungen der Formel (I) auftreten:

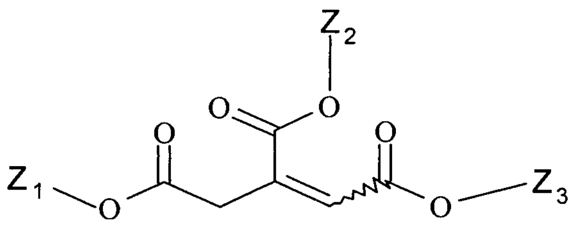

(III)

wobei

die Gruppen $Z_i$ ($Z_1$, $Z_2$ und $Z_3$) unabhängig voneinander verzweigte oder unverzweigte, Alkylgruppen oder Gruppen der Struktur $R_i(A(CR^1R^2)_{ci}(CR^3R^4)_{c'i})_{di}$- mit den jeweiligen Indizes ci und c'i unabhängig voneinander=0-10, bevorzugt 0-6, insbesondere 0-2, und di=1-5 sind, wobei $R_i$ ein verzweigter oder unverzweigter, fluorhaltiger Alkylrest ist, $R^1$ bis $R^4$ unabhängig voneinander Wasserstoff oder eine verzweigte oder unverzweigte Alkylgruppe sind, ci und c'i bevorzugt nicht gleichzeitig = 0 sind, A = O, S und/oder N ist und mindestens eine der Gruppen $Z_i$ eine Gruppe der Struktur $R_i(A(CR^1R^2)_{ci}(CR^3R^4)_{c'i})_{di}$- ist.

Bevorzugte Verbindungen der Formel (III) sind solche, in denen $Z_1$, $Z_2$ und $Z_3$ unabhängig voneinander $F_3C(CF_2)_{ai}(CH_2)_{bi}(O(CH_2)_{ci})_{di}$-Gruppen mit ai=0-6, insbesondere ai=1-5, bevorzugt 1-2, bi=1-6, bevorzugt 1-3, insbesondere bi=1-2, ci=2-10, insbesondere ci=2, und di=1-5 insbesondere di=1 sind. Besonders geeignet sind Verbindungen mit $Z_1$=$Z_2$=$Z_3$. Bevorzugt sind auch Verbindungen, in denen ai = 0, 1 oder 2, bevorzugt gleich 1 bis 2, insbesondere gleich 1, bi = 1, $c_i$ = 2 und $d_i$ = 1 oder 2, insbesondere gleich 1, ist und wobei $a_1 + a_2 + a_3 > 1$ ist.

Ein fünfter Gegenstand der vorliegenden Erfindung ist die Verwendung der Verbindungen der Formel (III) als Monomere oder Co-Monomere bei der Synthese fluorierter Polymere.

Die Offenbarungen in den zitierten Literaturstellen gehören hiermit ausdrücklich auch zum Offenbarungsgehalt der vorliegenden Anmeldung. Die folgenden Beispiele erläutern die vorliegende Erfindung näher, ohne den Schutzbereich zu beschränken.

## Beispiele

### Beispiel 1: Synthese des Sulfotricarballylats der Formel (Ia-1)

a) Veresterung

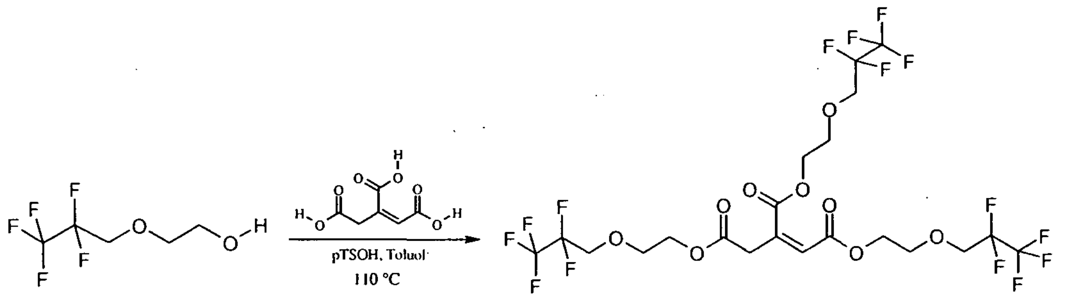


Der verwendete Alkohol wird wie in der Literatur beschrieben (Heilmann et al. J. Fluorine Chem. 1992, 59, 387; Janulis et al. US 5157159 (1992)) hergestellt. Anschließend wird ein Gemisch aus 72.4 mmol Alkohol, 18.1 mmol Aconitsäure (90 %ig, Alfa Aesar) und 3.6 mmol Toluol-4-sulfonsäure-Monohydrat (Merck KGaA) in 40 ml Toluol 48 Stunden unter Rückfluss gerührt. Das bei der Reaktion freiwerdende Wasser wird mit Hilfe eines Wasserabscheiders entfernt. Es wird mit Wasser gequenscht. Anschließend wird mit Toluol extrahiert und die vereinigten organischen Phasen mit Wasser gewaschen, über Natriumsulfat getrocknet und filtriert. Das Lösungsmittel wird am Rotationsverdampfer abdestilliert. Reinigung: Säulenchromatographisch über Silicagel;

Eluens: Toluol/Essigester 1/2
Substanz: $C_{21}H_{21}F_{15}O_9$; M=702.362 g/mol

$^1$H-NMR (400 MHz; in DMSO-$d_6$): 6.84 (s); 4.38-4.09 (m); 3.92-3.74 (m)ppm. $^{19}$F-NMR (376 MHz; in DMSO-$d_6$): -82.85 - -83.02 (m); -122.79 - -122.94 (m) ppm.
MS (EI, 70eV) m/z: 553 (10 %); 509 (10 %); 221 (15%); 177 (100%).

b) Addition

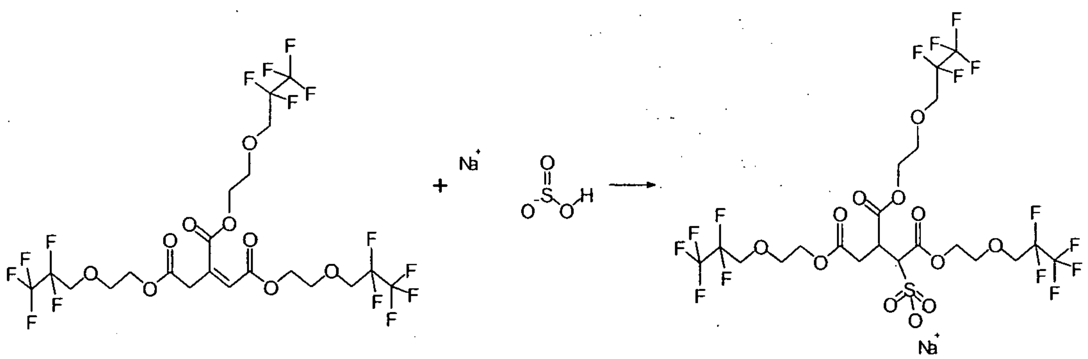

Zu einer Lösung aus 13.5 mmol des Triesters in 45 ml 1,4-Dioxan (Merck KGaA) werden bei 50 °C 68ml Natriumhydrogensulfit-Lösung (39 %ig in Wasser, Merck KGaA) (338 mmol) zugetropft und unter Rückfluss gerührt. Nach dem Zutropfen wird die Reaktionstemperatur für 65 Stunden bei 88 °C gehalten. Nach 24 Stunden und 48 Stunden

werden erneut je 23 ml Natriumhydrogensulfit-Lösung (39 %ig in Wasser, Merck KGaA) (113 mmol) zugetropft. Das Lösungsmittel wird am Rotationsverdampfer entfernt. Das Rohprodukt wird anschließend in Diethylether (Merck KGaA) aufgenommen, gerührt und filtriert. Der Filterrückstand wurde ein weiteres Mal ausgerührt und filtriert. Die Diethylether-Phasen wurden vereinigt und das organische Lösungsmittel am Rotationsverdampfer entfernt.
Substanz: $C_{21}H_{22}F_{15}O_{12}SNa$; M=806.424 g/mol
$^1$H-NMR (400 MHz; in DMSO-$d_6$): 4.31-4.06 (m); 4.00 (d); 3.95 (d); 3.88-3.71 (m); 3.68-3.54 (m); 3.48-3.37 (m); 3.36-3.00 (m); 2.92 (d); 2.87 (d) ppm. $^{19}$F-NMR (376 MHz; in DMSO-$d_6$): -83.23 - -83.40 (m); -123.16 - -123.55 (m) ppm.
MS (FIA-ESI) *m/z:*
Pos. Mode: 807 $(M + H)^+$
Neg. Mode: 783 $(M - Na)^-$

**Beispiel 2: Bestimmung der statischen Oberflächenspannung**

Es werden die statischen Oberflächenspannungen y von wässrigen Tensidlösungen mit verschiedenen Konzentrationen c (Gewichtsprozent) bestimmt.

Gerät: Tensiometer der Firma Sinterface (Modell PAT1)
Temperatur der Messlösungen: Raumtemperatur

Eingesetzte Messmethode: Messung der Oberflächenspannung am hängenden Tropfen gegen Luft. Hierbei werden die Hauptkrümmungsradien ($r_1$ und $r_2$) der Ellipsoiden (Tropfen) durch Tropfenkonturanalyse bestimmt. Da die Druckdifferenz ($\Delta$p) zwischen Außen- und Innenseite einer Grenzfläche indirekt proportional zu den Krümmungsradien ist, lässt sich die Oberflächenspannung über folgenden Zusammenhang berechnen:

$$\Delta p = \gamma\left(\frac{1}{r_1} + \frac{1}{r_2}\right)$$

Einstellungen am Gerät: Tropfenvolumina 7-10 $mm^3$ ; Messzeit 1500-3600s; 1,5 Bilder/s, Dichte des Tropfens= 1 $g/cm^3$.
Die Messwerte sind in der Tabelle 1 wiedergegeben. Die Abbildung 1 zeigt die statische Oberflächenspannung in Abhängigkeit von der Konzentration für das Sulfocarballylat gemäß Beispiel 1 b).

Tabelle 1

| c [%] | $\gamma$[mN/m] |
|---|---|
| 0,001 | 49,4 |
| 0,013 | 35,6 |
| 0,127 | 28,5 |
| 0,240 | 24,7 |
| 0,308 | 22,9 |
| 0,370 | 22,6 |
| 0,414 | 22,3 |
| 0,451 | 21,9 |
| 0,485 | 21,2 |
| 0,513 | 20,9 |
| 0,610 | 21,2 |
| 1,265 | 20,7 |

**Beispiel 3: Bestimmung der dynamischen Oberflächenspannung**

Es wird die dynamische Oberflächenspannung γ einer 0.1%igen (Gewichtsprozent) wässrigen Lösung der Verbindung 1 b) bestimmt. Eingesetzte Messmethode: Messung der Oberflächenspannung mit der Blasendruckmethode

Gerät: Tensiometer der Firma SITA (Modell t 60)
Temperatur der Messlösungen: 21 °C ± 0,2°C

Bei der Messung der dynamischen Oberflächenspannung werden Luftblasen mit verschiedenen Geschwindigkeiten durch eine Kapillare in die Tensidlösung gedrückt. Aus der dabei auftretenden Druckänderung kann die Oberflächenspannung in Abhängigkeit von der Blasenlebensdauer mit folgender Gleichung bestimmt werden:

$$\gamma = \frac{r(p_{max} - \rho \cdot g \cdot h)}{2}$$

$p_{max}$= Maximaldruck, ρ= Dichte der Flüssigkeit, h= Eintauchtiefe, r= Radius der Kapillare

Die Messwerte sind in der Tabelle 2 wiedergegeben. Die Abbildung 2 zeigt die dynamische Oberflächenspannung in Abhängigkeit von der Blasenlebensdauer für die Verbindung 1 b).

Tabelle 2

| Blasenlebensdauer **[ms]** | γ**[mN/m]** |
|---|---|
| 30 | 56,1 |
| 38 | 51,5 |
| 50 | 47,7 |
| 65 | 44,1 |
| 82 | 41,9 |
| 113 | 40 |
| 141 | 38,8 |
| 189 | 37,9 |
| 243 | 37,2 |
| 306 | 36,3 |
| 414 | 35,7 |
| 533 | 35,6 |
| 674 | 35,2 |
| 924 | 34,6 |
| 1299 | 34,2 |
| 1727 | 33,9 |
| 2200 | 33,7 |
| 2507 | 33,5 |
| 3204 | 33,5 |
| 4102 | 32,9 |
| 5473 | 32,9 |
| 7012 | 32,7 |
| 9132 | 32,5 |

(fortgesetzt)

| Blasenlebensdauer **[ms]** | **γ[mN/m]** |
|---|---|
| 11537 | 32,3 |
| 15707 | 32,1 |
| 19795 | 31,8 |
| 26529 | 31,5 |
| 35138 | 31,4 |
| 44790 | 31,3 |
| 58574 | 31 |

**Beispiel 4: Antikratertest in einem Polyurethan Wasserlack**

Ein Lack aus den Rohstoffen gemäß Tabelle 3 wird hergestellt, bei dem Oberflächendefekte (Krater) durch Überdosierung des Entschäumers BYK 023 gezielt erzeugt werden. Eine hochkonzentrierte Lösung (95 Gew.-%) des Sulfotricarballylats gemäß Beispiel 1 b in Dowanol PM (Dow Chemicals), wird in unterschiedlichen Mengen in den Lack eingearbeitet und hinsichtlich ihrer Wirksamkeit mit der 0-Probe (ohne Sulfotricarballylat) verglichen.

Tabelle 3:

| Rohstoffe für Lackrezeptur | | |
|---|---|---|
| Produkt | Funktion | Hersteller |
| Bayhydrol XP 2470 | Bindemittel | Bayer |
| Bayhydur 304 | PUR Härter | Bayer |
| Methoxybutylacetat | Lösemittel | VWR |
| BYK-023 | Entschäumer | BYK Chemie |
| Pigmentpaste blau | Kontrast zu Substrat | BASF |

Bei der Herstellung der kontaminierten Lackprobe wird zunächst Bindemittel, Pigmentpaste und Wasser vorgelegt (Tabelle 4a; PE Becher: 860ml) und anschließend eine Teilmenge der Vorlage komplettiert (Tabelle 4b; PE Becher 350 ml).

Tabelle 4a

| Vorgelegte Komponenten 1 | | | |
|---|---|---|---|
| Pos. | Rohstoff | Rezeptur [g] | Einstellung Dissolver/ Rührscheibe |
| 1 | Bayhydrol XP 2470 | 271,18 | 400 U/min; 10min; 80 mm |
| 2 | Pigmentpaste | 8 | |
| 3 | $H_2O$ | 27,28 | |

Tabelle 4b

| Komplettierung | | | |
|---|---|---|---|
| Pos. | Rohstoff | Rezeptur [g] | Einstellung Dissolver/ Rührscheibe |
| 4 | Vorlage | 128,55 | 400 U/min; 10min; 80 mm |
| 5 | Bayhydur 304 | 41,03 | |
| 6 | Methoxybutylacetat | 10,27 | |
| 7 | H2O | 19,62 | |
| 8 | BYK 023 | 0,14 | 600 U/min; 10min; 60 mm |

Zur Einarbeitung der Tensidlösung wird diese zunächst vorgelegt (PE Becher 175 ml) und anschließend die Lackmenge auf die Einwaage des Tensids bezogen. Es werden drei Testlacke mit unterschiedlichem Tensidgehalt bzw. ohne Tensid hergestellt (Tabelle 5).

Tabelle 5

| Einarbeitung der Tensidlösung | | | | |
|---|---|---|---|---|
| Lackprobe | Einwaage Tensidlösung [g] | Einwaage Lack [g] | C* Tensid[ %] | Einstellung Dissolver/ Rührscheibe |
| 1 | - | - | 0-Probe | - |
| 2 | 0,0544 | 37,42 | 0,06 | 900 U/min; 5min; 40 mm |
| 3 | 0,2064 | 47,39 | 0,19 | 900 U/min; 5min; 40 mm |
| * Gehalt Tensid bezogen auf Gesamtmenge der Lackprobe | | | | |

Die Lacke werden mit Hilfe eines automatischen Filmziehgeräts mit Vakuumansaugung (Byk Gardner E-2101) mit einem Filmziehrahmen (Erichson Model 360; Füllmenge: 4 ml Lack; Dosierung: 20 ml Spritzen; Ziehgeschwindigkeit 50 mm/s; Nassschichtdicken: 30 $\mu$m) auf weiße Lackkarten (219x286 mm; Hersteller: Leneta) aufgerakelt.

Die Lackprobe 2 ist nahezu, die Lackprobe 3 vollständig, frei von Oberflächendefekten (Kratern). Wohingegen die Lackprobe 1 zahlreiche Oberflächendefekte (Krater) zeigt.

Die Abbildungen 1 und 2 zeigen die statische bzw. die dynamische Oberflächenspannung für die Verbindung 1 b).

**Beispiel 5: Synthese des Sulfotricarballylats der Formel (Ia-2)**

Gemäß dem in Beispiel 1 beschriebenen Verfahren wird das Sulfotricarballylat der Formel (Ia-2) hergestellt.
Summenformel: $C_{24}H_{22}F_{21}O_{12}SNa$, M= 956.447 g/mol
$^1$H-NMR (300 MHz; in DMSO-$d_6$): 4.26-4.02 (m); 3.91 (dd); 3.82-3.67 (m); 3.63-3.46 (m); 3.44-3.30 (m); 3.25-2.98 (m); 2.89-2.77 (m) ppm. $^{19}$F-NMR (282 MHz; in DMSO-$d_6$): -80.42- -80.82 (m); -127.11- -127.50 (m); -119.79- -120.88 (m) ppm.
MS (FIA-ESI) *m/z:*
Neg. Mode: 933 $(M-Na)^-$

Anschließend wird sowohl die statische Oberflächenspannung wie in Beispiel 2 beschrieben als auch die dynamische Oberflächenspannung wie in Beispiel 3 beschrieben bestimmt. Die Ergebnisse sind in den Tabellen 6 und 7 wiedergegeben.

Tabelle 6

| c [%] | $\gamma$[mN/m] |
|---|---|
| 0,0001 | 54,2 |
| 0,0011 | 30,6 |
| 0,0018 | 27,8 |
| 0,0055 | 25,8 |
| 0,011 | 21,5 |

(fortgesetzt)

| c [%] | γ[mN/m] |
|---|---|
| 0,016 | 18,4 |
| 0,038 | 17,8 |
| 0,045 | 17,8 |
| 0,050 | 17,8 |
| 0,024 | 17,8 |
| 0,100 | 17,6 |
| 0,994 | 17,6 |

Tabelle 7

| Blasenlebensdauer [ms] | γ[mN/m] |
|---|---|
| 31 | 68 |
| 38 | 66,3 |
| 49 | 62,7 |
| 65 | 58,4 |
| 83 | 52,1 |
| 108 | 44,7 |
| 144 | 34,8 |
| 191 | 27,7 |
| 241 | 25 |
| 325 | 22,9 |
| 406 | 20,4 |
| 522 | 19,3 |
| 697 | 18,9 |
| 859 | 18,8 |
| 1163 | 18,3 |
| 1811 | 18,1 |
| 2250 | 17,8 |
| 2590 | 17,8 |
| 3223 | 17,6 |
| 4028 | 17,4 |
| 5615 | 17,6 |
| 7247 | 17,5 |
| 8998 | 17,4 |
| 12741 | 17,2 |
| 16566 | 17,5 |
| 19736 | 17,1 |
| 27035 | 17,3 |

(fortgesetzt)

| Blasenlebensdauer **[ms]** | **γ[mN/m]** |
|---|---|
| 34338 | 16,9 |
| 43556 | 16,8 |
| 56679 | 16,8 |

**Beispiel 6: Synthese des Sulfotricarballylats der Formel (Ia-3)**

Gemäß dem in Beispiel 1 beschriebenen Verfahren wird das Sulfotricarballylat der Formel (Ia-3) hergestellt.
Summenformel: $C_{21}H_{19}F_{18}O_{12}SNa$, M=860.395 g/mol
$^{1}$H-NMR (400 MHz; in DMSO-$d_6$): 5.48-5.37 (m); 5.35-5.21 (m); 4.30-4.09 (m); 4.07-3.90 (m); 3.57-3.46 (m); 3.40-3.32 (m); 3.15-3.05 (m); 2.82 (dd) ppm. $^{19}$F-NMR (376 MHz; in DMSO-$d_6$): -73.53- -73.85 (m) ppm.
MS (LC-ESI-MS negativ):
RT: 9.05 (837 $[M-H]^-$)

Anschließend wird sowohl die statische Oberflächenspannung wie in Beispiel 2 beschrieben als auch die dynamische Oberflächenspannung wie in Beispiel 3 beschrieben bestimmt. Die Ergebnisse sind in den Tabellen 8 und 9 wiedergegeben.

Tabelle 8

| **c [%]** | **γ[mN/m]** |
|---|---|
| 0,0001 | 57,9 |
| 0,001 | 52,5 |
| 0,01 | 44,0 |
| 0,10 | 29,2 |
| 0,25 | 25,4 |
| 0,53 | 22,7 |
| 1,03 | 21,7 |
| 1,22 | 21,3 |
| 2,46 | 21,0 |
| 4,74 | 20,8 |

Tabelle 9

| Blasenlebensd auer **[ms]** | **γ[mN/m]** |
|---|---|
| 57 | 31 |
| 53,1 | 38 |
| 47,8 | 52 |
| 44,7 | 64 |
| 42 | 85 |
| 39,8 | 111 |
| 38,7 | 144 |
| 37,4 | 190 |
| 36,5 | 243 |
| 36 | 324 |

(fortgesetzt)

| Blasenlebensd auer **[ms]** | γ**[mN/m]** |
|---|---|
| 35,5 | 423 |
| 35,2 | 512 |
| 34,9 | 706 |
| 34,6 | 938 |
| 34,4 | 1209 |
| 34 | 1773 |
| 33,8 | 2145 |
| 33,7 | 2498 |
| 33,6 | 3277 |
| 33,5 | 4172 |
| 33,2 | 5331 |
| 33,1 | 7431 |
| 33,1 | 9743 |
| 33,4 | 11981 |
| 32,7 | 15946 |
| 32,2 | 20560 |
| 32,1 | 27476 |
| 32,1 | 35812 |
| 32 | 47092 |
| 31,9 | 60416 |

**Beispiel 7: Synthese des Sulfotricarballylats der Formel (Ia-4)**

Gemäß dem in Beispiel 1 beschriebenen Verfahren wird das Sulfotricarballylat der Formel (Ia-4) hergestellt.
Summenformel: $C_{24}H_{16}F_{27}O_9SNa$, M=1016.391 g/mol
$^1$H-NMR (400 MHz; in DMSO-$d_6$): 4.51-4.25 (m); 4.14 (d); 4.08 (d); 3.70-3.59 (m); 3.55-3.45 (m); 3.37-3.15 (m); 2.97 (dd) ppm. $^{19}$F-NMR (376 MHz; in DMSO-$d_6$): -80.95 - -81-39 (m); -113.39 - -114.00 (m); -124.36 - -124.82 (m); -126.02 - -126.38 (m) ppm.
MS (LC-ESI-MS negativ):
RT: 10.60 (993 $[M-H]^-$)

Anschließend wird sowohl die statische Oberflächenspannung wie in Beispiel 2 beschrieben als auch die dynamische Oberflächenspannung wie in Beispiel 3 beschrieben bestimmt. Die Ergebnisse sind in den Tabellen 10 und 11 wiedergegeben.

Tabelle 10

| c [%] | γ[mN/m] |
|---|---|
| 0,0001 | 71,6 |
| 0,0003 | 66,3 |
| 0,0006 | 40,8 |
| 0,0010 | 22,4 |
| 0,0018 | 17,6 |
| 0,0020 | 17,5 |

(fortgesetzt)

| c [%] | γ[mN/m] |
|---|---|
| 0,0024 | 17,0 |
| 0,005 | 16,8 |
| 0,01 | 16,6 |
| 0,10 | 16,3 |
| 0,93 | 16,2 |

Tabelle 11

| Blasenlebensdauer [ms] | γ[mN/m] |
|---|---|
| 31 | 72,3 |
| 38 | 72,2 |
| 49 | 72,1 |
| 68 | 72,1 |
| 84 | 72,2 |
| 112 | 71,8 |
| 145 | 71,9 |
| 190 | 71,6 |
| 244 | 71,3 |
| 320 | 71,3 |
| 391 | 71 |
| 521 | 71 |
| 706 | 70,5 |
| 869 | 70,2 |
| 1224 | 70,4 |
| 1613 | 70 |
| 2102 | 70,3 |
| 2572 | 70,2 |
| 3329 | 69,5 |
| 4290 | 69,2 |
| 5506 | 68,5 |
| 6850 | 67,5 |
| 9319 | 65,7 |
| 11716 | 64,2 |
| 15108 | 60 |
| 19279 | 55,8 |
| 24035 | 50,2 |
| 30865 | 40,8 |
| 40016 | 32,1 |

(fortgesetzt)

| Blasenlebensdauer **[ms]** | γ**[mN/m]** |
|---|---|
| 55320 | 24,4 |

**Beispiel 8: Synthese des Sulfotricarballylats der Formel (Ia-5)**

Gemäß dem in Beispiel 1 beschriebenen Verfahren wird das Sulfotricarballylat der Formel (Ia-5) hergestellt.
Summenformel: $C_{30}H_{16}F_{39}O_9SNa$, M=1316.436 g/mol
$^1$H-NMR (300 MHz; in DMSO-$d_6$): 4.53-4.25 (m); 4.13 (d); 4.06 (d); 3.69-3.60 (m); 3.54-3.46 (m); 3.27 (ddd); 3.04-2.96 (m); 2.61-2.42 (m) ppm. $^{19}$F-NMR (282 MHz; in DMSO-$d_6$): -81.74 - -82.27 (m); -113.61 - -114.41 (m); -122.50 (s); -123.29 - -124.35 (m); -126.74 - -127.31 (m) ppm.
MS (LC-ESI-MS negativ):
RT: 11.91 (1293 $[M-H]^-$)

Anschließend wird sowohl die statische Oberflächenspannung wie in Beispiel 2 beschrieben als auch die dynamische Oberflächenspannung wie in Beispiel 3 beschrieben bestimmt. Die Ergebnisse sind in den Tabellen 12 und 13 wiedergegeben.

Tabelle 12

| **c [%]** | γ**[mN/m]** |
|---|---|
| 0,0001 | 71,9 |
| 0,001 | 71,5 |
| 0,01 | 71,4 |
| 0,10 | 52,9 |
| 0,98 | 54,5 |

Tabelle 13

| Blasenlebensdauer **[ms]** | γ**[mN/m]** |
|---|---|
| 31 | 72,8 |
| 38 | 72,9 |
| 49 | 72,8 |
| 64 | 72,8 |
| 86 | 72,9 |
| 110 | 72,7 |
| 146 | 72,7 |
| 187 | 72,9 |
| 244 | 72,9 |
| 319 | 73,1 |
| 393 | 73,2 |
| 524 | 73,3 |
| 715 | 73,4 |
| 890 | 73,3 |
| 1257 | 73,1 |
| 1614 | 72,9 |

(fortgesetzt)

| Blasenlebensdauer **[ms]** | **γ[mN/m]** |
|---|---|
| 2142 | 73 |
| 2536 | 73 |
| 3283 | 72,8 |
| 4222 | 72,8 |
| 5476 | 72,8 |
| 7128 | 72,9 |
| 9188 | 72,7 |
| 11868 | 72,7 |
| 15248 | 72,5 |
| 20919 | 72,5 |
| 26720 | 72,3 |
| 34303 | 72,3 |
| 44207 | 72,1 |
| 60491 | 72,2 |

**Beispiel 9: Synthese des Sulfotricarballylats der Formel (Ia-6)**

Gemäß dem in Beispiel 1 beschriebenen Verfahren wird das Sulfotricarballylat der Formel (Ia-6) hergestellt.
Summenformel: $C_{30}H_{22}F_{33}O_{12}SNa$, M=1256.492 g/mol
$^{1}$H-NMR (400 MHz; in DMSO-$d_6$): 4.28-4.05 (m); 3.91 (d); 3.84 (d); 3.82-3.69 (m); 3.51-3.45 (m); 3.39-3.30 (m); 3.25-3.09 (m); 3.02 (dd); 2.83 (dd) ppm. $^{19}$F-NMR (376 MHz; in DMSO-$d_6$): -80.94 - -81.23 (m); -119.39 - -119.96 (m); -123.34 - -123.87 (m); -126.30 - - 126.76 (m) ppm.
MS (LC-ESI-MS negativ):
RT: 11.38 (1233 $[M-H]^-$)

Anschließend wird sowohl die statische Oberflächenspannung wie in Beispiel 2 beschrieben als auch die dynamische Oberflächenspannung wie in Beispiel 3 beschrieben bestimmt. Die Ergebnisse sind in den Tabellen 14 und 15 wiedergegeben.

Tabelle 14

| **c [%]** | **γ[mN/m]** |
|---|---|
| 0,0001 | 72,2 |
| 0,0011 | 72,1 |
| 0,0020 | 65,0 |
| 0,0023 | 60,6 |
| 0,0033 | 44,0 |
| 0,0038 | 26,8 |
| 0,0050 | 15,8 |
| 0,0100 | 15,7 |
| 0,10 | 15,4 |
| 1,00 | 15,4 |

Tabelle 15

| Blasenlebensdauer **[ms]** | $\gamma$**[mN/m]** |
|---|---|
| 31 | 71,7 |
| 40 | 71,4 |
| 51 | 71,4 |
| 64 | 70,9 |
| 84 | 70,9 |
| 111 | 70,9 |
| 144 | 70,4 |
| 186 | 70,4 |
| 244 | 70,1 |
| 322 | 69,8 |
| 398 | 69,7 |
| 525 | 69,6 |
| 715 | 69,5 |
| 934 | 68,7 |
| 1221 | 67,7 |
| 1597 | 67,2 |
| 2099 | 66 |
| 2501 | 65,3 |
| 3243 | 64,3 |
| 4142 | 62,3 |
| 5208 | 61 |
| 7135 | 58,1 |
| 8938 | 55,7 |
| 11481 | 53,5 |
| 15165 | 50,3 |
| 19619 | 48,2 |
| 24941 | 46,1 |
| 33879 | 44 |
| 41071 | 41,8 |
| 54632 | 40,7 |

## Patentansprüche

1. Verbindungen der Formel (I)

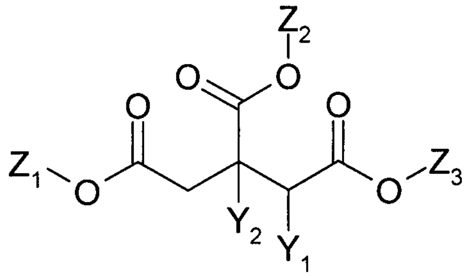

X

(I)

wobei

die Gruppen $Z_i$ ($Z_1$, $Z_2$ und $Z_3$) unabhängig voneinander verzweigte oder unverzweigte Alkylgruppen oder Gruppen der Struktur $R_i(A(CR^1R^2)_{ci}(CR^3R^4)_{c'i})_{di}$- mit den jeweiligen Indizes ci und c'i unabhängig voneinander=0-10 und di=1-5 sind, wobei $R_i$ ein verzweigter oder unverzweigter, fluorhaltiger Alkylrest ist, $R^1$ bis $R^4$ unabhängig voneinander Wasserstoff oder eine verzweigte oder unverzweigte Alkylgruppe sind, ci und c'i nicht gleichzeitig = 0 sind und A = O, S und /oder N ist,
$Y_1$ eine anionische polare Gruppe und $Y_2$ ein Wasserstoffatom ist oder umgekehrt,
X ein Kation ist
und mindestens eine der Gruppen $Z_i$ eine Gruppe der Struktur $R_i(A(CR^1R^2)_{ci}(CR^3R^4)_{c'i})_{di}$- ist.

2. Verbindungen gemäß Anspruch 1, **dadurch gekennzeichnet, dass** die Variablen der Formel (I) die folgenden Bedeutungen haben:

die Gruppen $Z_i$ ($Z_1$, $Z_2$ und $Z_3$) sind unabhängig voneinander verzweigte oder unverzweigte Alkylgruppen oder Gruppen der Struktur $R_i(O(CH_2)_{ci})_{di}$ mit den jeweiligen Indizes ci=2-10 und di=1-5, wobei $R_i$ ein verzweigter oder unverzweigter, fluorhaltiger Alkylrest ist,
$Y_1$ ist eine anionische polare Gruppe und $Y_2$ ist ein Wasserstoffatom oder umgekehrt,
X ist ein Kation
und mindestens eine der Gruppen $Z_i$ ist eine Gruppe der Struktur $R_i(O(CH_2)_{ci})_{di}$ ist.

3. Verbindungen gemäß Anspruch 1 oder 2, **dadurch gekennzeichnet, dass** die nichtfluorierten, verzweigten oder unverzweigten Alkylgruppen 1 bis 10, insbesondere 3 bis 8, Kohlenstoffatome enthalten.

4. Verbindungen gemäß einem oder mehreren der Ansprüche 1 bis 3, **dadurch gekennzeichnet, dass** X ein einwertiges Kation ist, insbesondere $H^+$ oder ein Alkalimetall-Kation, insbesondere $Na^+$, oder $NR_4^+$, wobei R = H oder C1 - C6-Alkyl, insbesondere $H^+$, ist und alle R gleich oder verschieden sein können

5. Verbindungen gemäß einem oder mehreren der Ansprüche 1 bis 4, **dadurch gekennzeichnet, dass** die Verbindungen zwei oder drei, insbesondere drei, fluorierte Gruppen $Z_i$ enthalten.

6. Verbindungen gemäß einem oder mehreren der Ansprüche 1 bis 5, **dadurch gekennzeichnet, dass** $Z_1$, $Z_2$ und $Z_3$ unabhängig voneinander $F_3C(CF_2)_{ai}(CH_2)_{bi}(O(CH_2)_{ci})_{di}$-Gruppen mit ai=0-2, insbesondere ai=1-2, bi=1-6, insbesondere bi=1-2, ci=2-10, insbesondere ci=2, und di=1-5, insbesondere di=1 sind, wobei insbesondere $Z_1$=$Z_2$=$Z_3$ sind.

7. Verbindungen gemäß einem oder mehreren der Ansprüche 1 bis 6, **dadurch gekennzeichnet, dass** $Y_1$ oder $Y_2$, insbesondere $Y_1$, eine Sulfonatgruppe $-SO_3^-$ ist.

8. Verbindungen gemäß einem oder mehreren der Ansprüche 1 bis 7, **dadurch gekennzeichnet, dass** $Z_1$=$Z_2$=$Z_3$= $F_3C(CF_2)_{ai}(CH_2)_{bi}(O(CH_2)_{ci})_{di}$, mit ai=1-2, bi=1-2, ci=2 und di=1, $Y_1$ eine Sulfonatgruppe $-SO_3^-$, $Y_2$ ein Wasserstoffatom und X=$Na^+$ ist.

9. Verbindungen gemäß einem oder mehreren der Ansprüche 1 bis 8, **dadurch gekennzeichnet, dass** sie den Formeln (Ia) oder (Ib) entsprechen

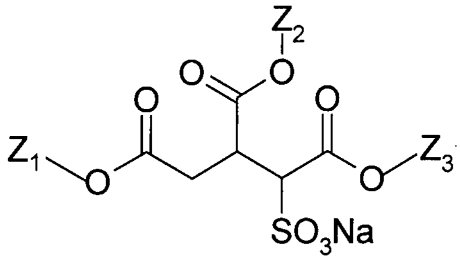

(Ia)

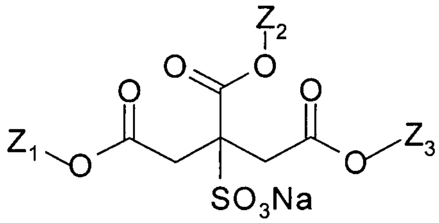

(Ib)

wobei $Z_1=Z_2=Z_3$ und alle $Z_i$ ausgewählt sind aus $R_i(O(CH_2)_{ci})_{di}$ mit ci=2, di=1-3 und $R_i$ = $CF_3CF_2CH_2$- oder $CF_3CF_2CF_2CH_2$-.

**10.** Verwendung von Verbindungen gemäß einem oder mehreren der Ansprüche 1 bis 9 als oberflächenaktives Mittel, insbesondere als Additive in Farb- und Lackzubereitungen, Druckfarben und Wachsen, sowie Poliermitteln.

**11.** Mittel, insbesondere Farb- und Lackzubereitungen, enthaltend mindestens eine Verbindung gemäß Anspruch 1 und einen für den jeweiligen Verwendungszweck geeigneten Träger sowie ggf. weitere spezifische Aktivstoffe.

**12.** Verfahren zur Herstellung von Verbindungen gemäß einem oder mehreren der Ansprüche 1 bis 9 umfassend a) die Veresterung von Aconitsäure oder Zitronensäure bzw. deren Anhydrid oder Säurechlorid mit einem oder mehreren Alkoholen der Formel $Z_iOH$ (II), bevorzugt in Gegenwart eines Katalysators, und b) die Addition, bevorzugt von Natriumhydrogensulfit, an die olefinische Doppelbindung bzw. Derivatisierung der OH-Gruppe, wobei die Alkohole Gruppen $Z_i$ gemäß Anspruch 1 enthalten.

**13.** Verbindungen der Formel (III)

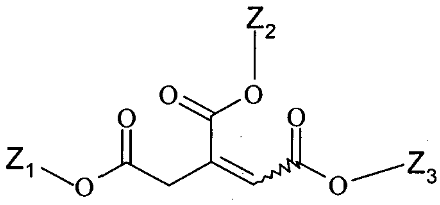

(III)

wobei

die Gruppen $Z_i$ ($Z_1$, $Z_2$ und $Z_3$) unabhängig voneinander verzweigte oder unverzweigte, Alkylgruppen oder Gruppen der Struktur $R_i(A(CR^1R^2)_{ci}(CR^3R^4)_{c'i})_{di}$- mit den jeweiligen Indizes ci und c'i unabhängig voneinander = 0-10 und di=1-5 sind, wobei $R_i$ ein verzweigter oder unverzweigter, fluorhaltiger Alkylrest ist, $R^1$ bis $R^4$ unabhängig voneinander Wasserstoff oder eine verzweigte oder unverzweigte Alkylgruppe sind und A = O, S und/oder N ist,
und mindestens eine der Gruppen $Z_i$ eine Gruppe der Struktur $R_i(A(CR^1R^2)_{ci}(CR^3R^4)_{c'i})_{di}$-ist und ci und c'i nicht gleichzeitig = 0 sind.

**14.** Verbindungen gemäß Anspruch 13, **dadurch gekennzeichnet, dass** $Z_1$, $Z_2$ und $Z_3$ unabhängig voneinander $F_3C(CF_2)_{ai}(CH_2)_{bi}(O(CH_2)_{ci})_{di}$-Gruppen mit ai=0-6, insbesondere ai=1-2, bi=1-6, insbesondere bi=1-2, ci=2-10, insbesondere ci=2, und di=1-5, insbesondere di=1-3 sind, wobei insbesondere $Z_1=Z_2=Z_3$ sind.

**15.** Verwendung von Verbindungen gemäß Anspruch 13 als Monomere oder Co-Monomere bei der Synthese fluorierter Polymere.

## Claims

**1.** Compounds of the formula (I)

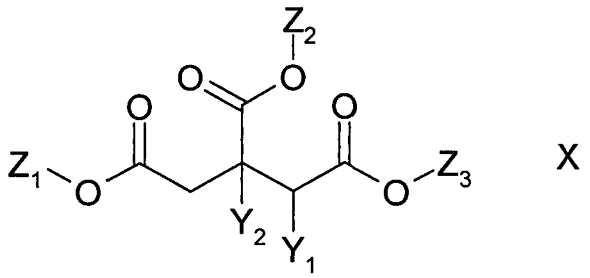

(I)

where

the groups $Z_i$ ($Z_1$, $Z_2$ and $Z_3$) are, independently of one another, branched or unbranched alkyl groups or groups of the structure $R_i(A(CR^1R^2)_{ci}(CR^3R^4)_{c'i})_{di}$-, where the respective indices ci and c'i are, independently of one another, =0-10 and di=1-5, where $R_i$ is a branched or unbranched, fluorine-containing alkyl radical, $R^1$ to $R^4$ are, independently of one another, hydrogen or a branched or unbranched alkyl group, ci and c'i are not simultaneously = 0, and A = O, S and/or N,
$Y_1$ is an anionic polar group and $Y_2$ is a hydrogen atom, or vice versa,
X is a cation,
and at least one of the groups $Z_i$ is a group of the structure $R_i(A(CR^1R^2)_{ci}(CR^3R^4)_{ci})_{di}$-.

**2.** Compounds according to Claim 1, **characterised in that** the variables in the formula (I) have the following meanings:

the groups $Z_i$ ($Z_1$, $Z_2$ and $Z_3$) are, independently of one another, branched or unbranched alkyl groups or groups of the structure $R_i(O(CH_2)_{ci})_{di}$, where the respective indices ci=2-10 and di=1-5, where $R_i$ is a branched or unbranched, fluorine-containing alkyl radical,
$Y_1$ is an anionic polar group and $Y_2$ is a hydrogen atom, or vice versa,
X is a cation,
and at least one of the groups $Z_i$ is a group of the structure $R_i(O(CH_2)_{ci})_{di}$.

**3.** Compounds according to Claim 1 or 2, **characterised in that** the unfluorinated, branched or unbranched alkyl groups contain 1 to 10, in particular 3 to 8, carbon atoms.

**4.** Compounds according to one or more of Claims 1 to 3, **characterised in that** X is a monovalent cation, in particular $H^+$, or an alkali metal cation, in particular $Na^+$, or $NR_4^+$, where R = H or C1 - C6-alkyl, in particular $H^+$, and all R may be identical or different.

**5.** Compounds according to one or more of Claims 1 to 4, **characterised in that** the compounds contain two or three, in particular three, fluorinated groups $Z_i$.

**6.** Compounds according to one or more of Claims 1 to 5, **characterised in that** $Z_1$, $Z_2$ and $Z_3$ are, independently of one another, $F_3C(CF_2)_{ai}(CH_2)_{bi}(O(CH_2)_{ci})_{di}$- groups, where ai=0-2, in particular ai=1-2, bi=1-6, in particular bi=1-2, ci=2-10, in particular ci=2, and di=1-5, in particular di=1, where, in particular, $Z_1=Z_2=Z_3$.

**7.** Compounds according to one or more of Claims 1 to 6, **characterised in that** $Y_1$ or $Y_2$, in particular $Y_1$, is a sulfonate group $-SO_3^-$.

**8.** Compounds according to one or more of Claims 1 to 7, **characterised in that** $Z_1=Z_2=Z_3=F_3C(CF_2)_{ai}(CH_2)_{bi}(O(CH_2)_{ci})_{di}$, where ai=1-2, bi=1-2, ci=2 and di=1, $Y_1$ is a sulfonate group $-SO_3^-$, $Y_2$ is a hydrogen atom, and $X=Na^+$.

**9.** Compounds according to one or more of Claims 1 to 8, **characterised in that** they conform to the formula (Ia) or (Ib)

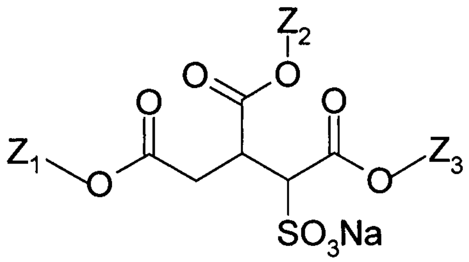

(Ia)

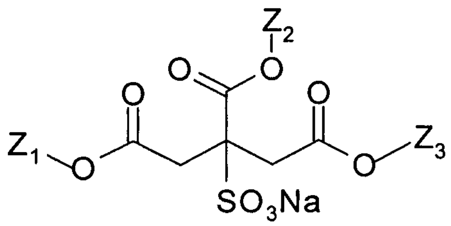

(Ib)

where $Z_1=Z_2=Z_3$ and all $Z_i$ are selected from $R_i(O(CH_2)_{ci})_{di}$, where ci=2, di=1-3 and $R_i=CF_3CF_2CH_2$- or $CF_3CF_2CF_2CH_2$-.

**10.** Use of compounds according to one or more of Claims 1 to 9 as surface-active agents, in particular as additives in paint and coating preparations, printing inks and waxes, as well as polishes.

**11.** Compositions, in particular paint and coating preparations, comprising at least one compound accoding to Claim1 and a vehicle which is suitable for the particular application, and optionally further specific active substances.

**12.** Process for the preparation of compounds according to one or more of Claims 1 to 9, comprising a) the esterification of aconitic acid or citric acid or the anhydride or acid chloride thereof using one or more alcohols of the formula $Z_iOH$ (II), preferably in the presence of a catalyst, and b) the addition, preferably of sodium hydrogensulfite, onto the olefinic double bond or derivatisation of the OH group, where the alcohols contain groups $Z_i$ according to Claim 1.

**13.** Compounds of the formula (III)

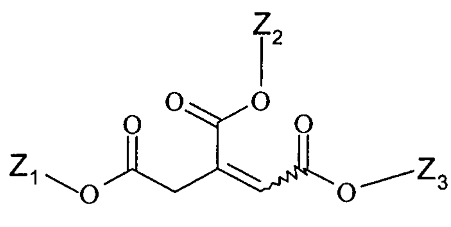

(III)

where

the groups $Z_i$ ($Z_i$, $Z_2$ and $Z_3$) are, independently of one another, branched or unbranched alkyl groups or groups of the structure $R_i(A(CR^1R^2)_{ci}(CR^3R^4)_{c'i})_{di}$-, where the respective indices ci and c'i are, independently of one another, = 0-10 and di=1-5, where $R_i$ is a branched or unbranched, fluorine-containing alkyl radical, $R^1$ to $R^4$ are, independently of one another, hydrogen or a branched or unbranched alkyl group, and A = O, S and/or N, and at least one of the groups $Z_i$ is a group of the structure $R_i(A(CR^1R^2)_{ci}(CR^3R^4)_{c'i})_{di}$-, and ci and c'i are not

simultaneously = 0.

14. Compounds according to Claim 13, **characterised in that** $Z_1$, $Z_2$ and $Z_3$ are, independently of one another, $F_3C(CF_2)_{ai}(CH_2)_{bi}(O(CH_2)_{ci})_{di}$-groups, where ai=0-6, in particular ai=1-2, bi=1-6, in particular bi=1-2, ci=2-10, in particular ci=2, and di=1-5, in particular di=1-3, where, in particular, $Z_1$=$Z_2$=$Z_3$.

15. Use of compounds according to Claim 13 as monomers or comonomers in the synthesis of fluorinated polymers.

## Revendications

1. Composés de formule (I)

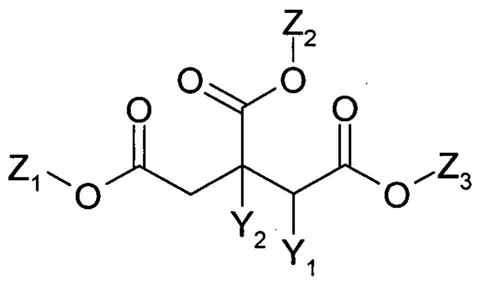

X

(I)

dans laquelle

les groupements $Z_i$ ($Z_1$, $Z_2$ et $Z_3$) sont, indépendamment les uns des autres, des groupements alkyle ramifiés ou non ramifiés ou des groupements de structure $R_i(A(CR^1R^2)_{ci}(CR^3R^4)_{c'i})_{di}$-, où les indices respectifs ci et c'i sont, indépendamment l'un de l'autre, =0-10 et di=1-5, où $R_i$ est un radical alkyle fluoré ramifié ou non ramifié, $R^1$ à $R^4$ sont, indépendamment les uns des autres, hydrogène ou un groupement alkyle ramifié ou non ramifié, ci et c'i ne valent pas simultanément = 0, et A = O, S et/ou N,
$Y_1$ est un groupement polaire anionique et $Y_2$ est un atome d'hydrogène, ou vice versa,
X est un cation,
et au moins l'un parmi les groupements $Z_i$ est un groupement de structure $R_i(A(CR^1R^2)_{ci}(CR_3R^4)_{c'i})_{di}$-.

2. Composés selon la revendication 1, **caractérisés en ce que** les variables de la formule (I) revêtent les significations suivantes :

les groupements $Z_i$ ($Z_1$, $Z_2$ et $Z_3$) sont, indépendamment les uns des autres, des groupements alkyle ramifiés ou non ramifiés ou des groupements de structure $R_i(O(CH_2)_{ci})_{di}$, où les indices respectifs ci=2-10 et di=1-5, où $R_i$ est un radical alkyle fluoré ramifié ou non ramifié,
$Y_1$ est un groupement polaire anionique et $Y_2$ est un atome d'hydrogène, ou vice versa,
X est un cation,
et au moins l'un parmi les groupements $Z_i$ est un groupement de structure $R_i(O(CH_2)_{ci})_{di}$.

3. Composés selon la revendication 1 ou 2, **caractérisés en ce que** les groupements alkyle non fluorés, ramifiés ou non ramifiés contiennent de 1 à 10, en particulier de 3 à 8, atomes de carbone.

4. Composés selon l'une ou plusieurs parmi les revendications 1 à 3, **caractérisés en ce que** X est un cation monovalent, en particulier $H^+$, ou un cation de métal alcalin, en particulier $Na^+$, ou $NR_4^+$, où R = H ou C1 - C6-alkyle, en particulier $H^+$, et tous les R peuvent être identiques ou différents.

5. Composés selon l'une ou plusieurs parmi les revendications 1 à 4, **caractérisés en ce que** les composés contiennent deux ou trois, en particulier trois, groupements fluorés $Z_i$.

6. Composés selon l'une ou plusieurs parmi les revendications 1 à 5, **caractérisés en ce que** $Z_1$, $Z_2$ et $Z_3$ sont, indépendamment les uns des autres, des groupements $F_3C(CF_2)_{ai}(CH_2)_{bi}(O(CH_2)_{ci})_{di}$-, où ai=0-2, en particulier

ai=1-2, bi=1-6, en particulier bi=1-2, ci=2-10, en particulier ci=2, et di=1-5, en particulier di=1, où, en particulier, $Z_1$=$Z_2$=$Z_3$.

**7.** Composés selon l'une ou plusieurs parmi les revendications 1 à 6, **caractérisés en ce que** $Y_1$ ou $Y_2$, en particulier $Y_1$, est un groupement sulfonate $-SO_3^-$.

**8.** Composés selon l'une ou plusieurs parmi les revendications 1 à 7, **caractérisés en ce que** $Z_1$=$Z_2$=$Z_3$=$F_3C(CF_2)_{ai}(CH_2)_{bi}(O(CH_2)_{ci})_{di}$, où ai=1-2, bi=1-2, ci=2 et di=1, $Y_1$ est un groupement sulfonate $-SO_3^-$, $Y_2$ est un atome d'hydrogène, et $X=Na^+$.

**9.** Composés selon l'une ou plusieurs parmi les revendications 1 à 8, **caractérisés en ce qu'**ils correspondent à la formule (Ia) ou (Ib)

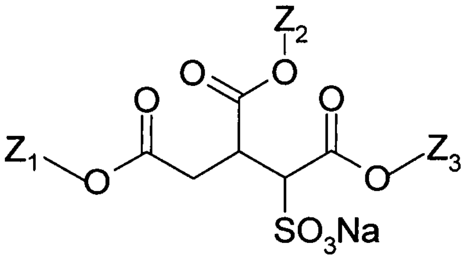

(Ia)

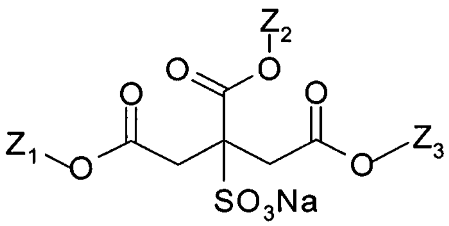

(Ib)

dans laquelle $Z_1$=$Z_2$=$Z_3$ et tous les $Z_i$ sont choisis parmi $R_i(O(CH_2)_{ci})_{di}$, où ci=2, di=1-3 et $R_i$=$CF_3CF_2CH_2$- ou $CF_3CF_2CF_2CH_2$-.

**10.** Utilisation de composés selon l'une ou plusieurs parmi les revendications 1 à 9 comme agents tensioactifs, en particulier comme adjuvants dans des préparations de peinture et de revêtement, des encres d'impression et des cires, ainsi que des produits à polir.

**11.** Compositions, en particulier des préparations de peinture et de revêtement, comprenant au moins un composé selon la revendication 1 et un véhicule convenable pour l'application particulière, et éventuellement d'autres substances actives spécifiques.

**12.** Procédé de préparation de composés selon l'une ou plusieurs parmi les revendications 1 à 9, comprenant a) l'estérification d'acide aconitique ou d'acide citrique ou de l'anhydride ou du chlorure d'acide de ceux-ci en utilisant un ou plusieurs alcools de formule $Z_iOH$ (II), de préférence en présence d'un catalyseur, et b) l'addition, de préférence d'hydrogénosulfite de sodium, à la double liaison ou la dérivatisation du groupement OH, où les alcools contiennent des groupements $Z_i$ selon la revendication 1.

**13.** Composés de formule (III)

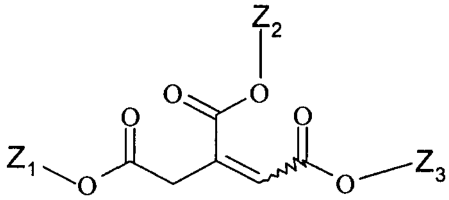

(III)

dans laquelle

les groupements $Z_i$ ($Z_1$, $Z_2$ et $Z_3$) sont, indépendamment les uns des autres, des groupements alkyle ramifiés ou non ramifiés ou des groupements de structure $R_i(A(CR^1R^2)_{ci}(CR^3R^4)_{c'i})_{di}$-, où les indices respectifs ci et c'i sont, indépendamment l'un de l'autre, = 0-10 et di=1-5, où $R_i$ est un radical alkyle fluoré ramifié ou non ramifié, $R^1$ à $R^4$ sont, indépendamment les uns des autres, hydrogène ou un groupement alkyle ramifié ou non ramifié, et A = O, S et/ou N,
et au moins l'un parmi les groupements $Z_i$ est un groupement de structure $R_i(A(CR^1R^2)_{ci}(CR^3R^4)_{c'i})_{di}$-, et ci et c'i ne valent pas simultanément = 0.

**14.** Composés selon la revendication 13, **caractérisés en ce que** $Z_1$, $Z_2$ et $Z_3$ sont, indépendamment les uns des autres, des groupements $F_3C(CF_2)_{ai}(CH_2)_{bi}(O(CH_2)_{ci})_{di}$-, où ai=0-6, en particulier ai=1-2, bi=1-6, en particulier bi=1-2, ci=2-10, en particulier ci=2, et di=1-5, en particulier di=1-3, où, en particulier, $Z_1=Z_2=Z_3$.

**15.** Utilisation de composés selon la revendication 13, comme monomères ou co-monomères dans la synthèse de polymères fluorés.

Abbildung 1: statische Oberflächenspannung γ des Sulfotricarballylats gemäß Beispiel 1b)

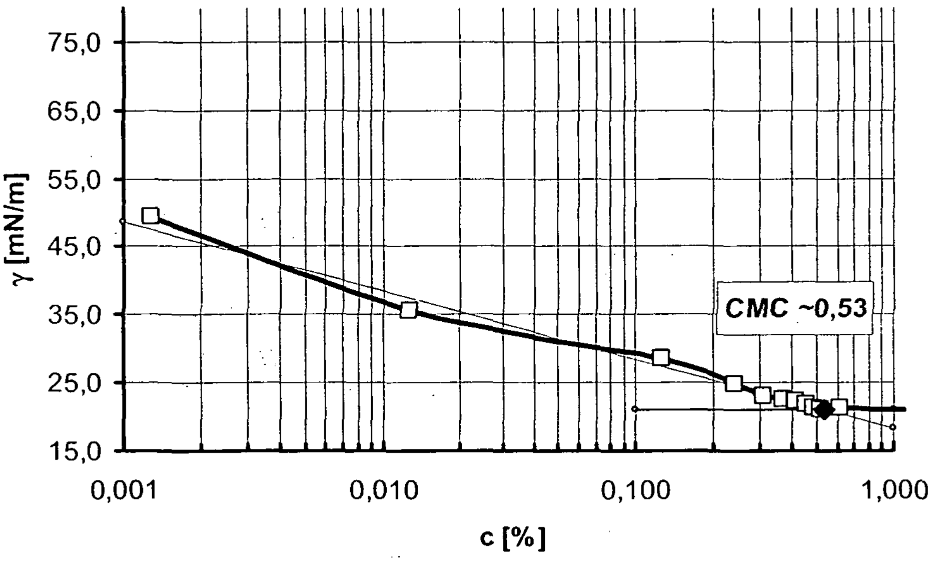


Abbildung 2: dynamischen Oberflächenspannungen γ des Sulfotricarballylats gemäß Beispiel 1b)

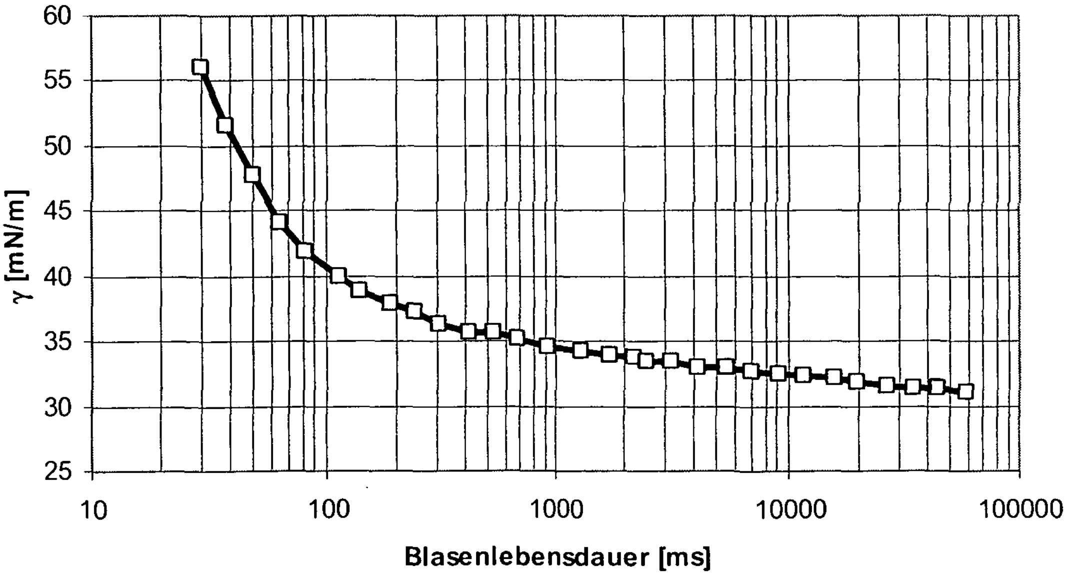

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4988610 A
- US 6890608 B
- US 5300394 A
- WO 03010128 A
- JP 2001133984 A
- JP 9111286 A
- DE 102005000858
- US 51571591992 A, Janulis


### In der Beschreibung aufgeführte Nicht-Patentliteratur


- **G. L. KENNEDY, JR. ; J. L. BUTENHOFF ; G. W. OLSEN ; J. C. O'CONNOR ; A. M. SEACAT ; R. G. BIEGEL ; S. R. MURPHY ; D. G. FARRAR.** *Critical Review in Toxicology,* 2004, vol. 34, 351-384
- **A.R. PITT et al.** *Colloids and Surfaces A: Physicochemical and Engineering Aspects,* 1996, vol. 114, 321-335
- **A.R. PITT.** *Progr. Colloid Polym. Sci,* 1997, vol. 103, 307-317
- **Z.-T. LIU et al.** *Ind. Eng. Chem. Res.,* 2007, vol. 46, 22-28
- **HEILMANN et al.** *J. Fluorine Chem.,* 1992, vol. 59, 387